# EUROPEAN PATENT SPECIFICATION

(11) **EP 3 046 500 B1**
(45) Date of publication and mention of the grant of the patent: **26.08.2020**
(21) Application number: 14854336.6
(22) Date of filing: 15.10.2014
(51) Int. Cl.: A61B 90/11, A61B 6/12, A61B 17/34, A61B 90/10, A61B 90/00, A61B 34/20, A61B 34/00

(54) **SURGICAL NAVIGATION SYSTEM AND RELATED DEVICE**
CHIRURGISCHES NAVIGATIONSSYSTEM UND ENTSPRECHENDE VORRICHTUNG
SYSTÈME CHIRURGICAL DE NAVIGATION ET DISPOSITIF ASSOCIÉ

(30) Priority: 16.10.2013 US 201361891661 P
(43) Date of publication of application: 27.07.2016
(73) Proprietor: ClearPoint Neuro, Inc., Irvine, CA 92618 (US)
(72) Inventor: PIFERI, Peter, Orange, California 92867 (US); PANDEY, Rajesh, Irvine, California 92604 (US); DALY, Maxwell Jerad, Redlands, California 92373 (US); JENKINS, Kimble L., Memphis, Tennessee 38111 (US)
(74) Representative: Yeadon IP Limited
(86) International application number: PCT/US2014/060644
(87) International publication number: WO 2015/057807

(56) References cited:
- WO-A1-2012/072112
- US-A1- 2007 129 629
- US-A1- 2008 214 922
- US-A1- 2009 112 084
- US-A1- 2009 171 184
- US-A1- 2010 160 771
- US-A1- 2012 046 542
- US-B1- 6 529 765
- US-B2- 6 920 347
- US-B2- 8 238 631

## Description

### FIELD OF THE INVENTION

The present invention relates to surgical navigation systems or tools for placement/localization of interventional medical devices and/or therapies in the body. Embodiments of the present invention may be particularly suitable for placing neuromodulation leads, such as Deep Brain Stimulation ("DBS") leads, placing implantable parasympathetic or sympathetic nerve chain leads and/or CNS stimulation leads and/or for delivering therapies to target internal locations, including intracranial locations, in the body.

### BACKGROUND OF THE INVENTION

Conventional, so-called image guided systems (IGS), also referred to as surgical navigation systems, are becoming more common and widely adapted in neurosurgery. Examples include, for example, navigation systems that use electromagnetic sensors or cameras with optical fiducials for tracking and registering tools and patients from an imaging space to a surgical space. Currently, many IGS systems are based on obtaining a pre-operative series of imaging data, such as, e.g., MRI and CT images which are registered to the patient in the physical world or surgical space and which include tracking systems for navigation during surgery. Optical tracking allows for detecting markers placed on a patient's skin and/or and surgical tools (known as "fiducials") using a camera for registration between an imaging space and a surgical space. Electromagnetic ("EM") tracking systems are also used for surgical navigation. *See, e.g.,* US Patent Nos. 7,706,600; 7,491,198; and 8,238,631 and US Patent Application Publications 2004/075768; 2012/0330135; and 2013/0060146. An MRI-surgical system having a trajectory guide with a yoke, a platform and a mounting column is shown in US 2009/171184 A1 by Jenkins et al, and also in US 2009/112084 by Piferi et al..

### SUMMARY

The invention is defined in the claims.

In particular, the present invention relates to a trajectory frame (100) for use with a surgical system, comprising: a base (110) having a patient access aperture (112) formed therein, wherein the base is configured to be secured to a body of a patient; a yoke (120) movably mounted to the base and rotatable about a roll axis; a platform (130) movably mounted to the yoke and rotatable about a pitch axis; an elongated guide (1102) secured to the platform, wherein the guide comprises opposite proximal (1102a) and distal (1102b) end portions, wherein the guide distal end portion is positioned proximate the patient access aperture, wherein the guide comprises a bore therethrough that extends from the proximal end portion to the distal end portion, and at least one device received by the guide (1102) within the bore, wherein the at least one device is removably secured to the guide, characterised in that the at least one device that is received within the bore is an elongate tracking probe mount (1160; 1190m) holding a tracking probe (1162; 1190) with reflective members (1164; 1194) arranged in a fixed geometric relationship relative to each other, and wherein the reflective members are configured to be detectable by a camera-based tracking system (10N/10T), wherein the tracking probe mount (1160; 1190m) and the trajectory frame (100) comprise image fiducials (50F) detectable in CT and/or MRI images to thereby allow a circuit to calculate an intrabody trajectory using two different methods, one calculated using image interrogation of images with the image fiducials and another calculated using a camera-based system with the reflective members.

Furthermore, the present invention relates to a surgical navigation system with a workstation having a User Interface (UI) (44) and a circuit (40) configured to provide a planned trajectory line on an image on a display using the trajectory frame as detailed above, with reflective members (1164; 1194) detectable by a camera (10C) of a camera-based navigation system (10N/10T) held by the trajectory frame, the circuit UI being configured to provide a user-selectable feature to allow a user to select a second calculation of a planned trajectory line calculated using CT and/or MRI image detectable fiducials (50F) held by or on the trajectory frame to confirm that the planned trajectory line from the camera is proper. Some aspects are directed to devices, interfaces, circuits, methods and systems that can work with radiation-based imaging modalities to facilitate precise trajectories, entry points and/or reduce radiation exposure by reducing the number and/or physical extent of images obtained during a respective image-guided and/or surgical navigation procedure and/or by allowing an independent calculation of trajectory or planned entry to a target site using the imaging modality and at least one tool where registration is not required.

In some aspects, concordance of the calculated trajectory using the conventional system with a calculated trajectory using the supplemental system can facilitate precise placement.

Some aspects are directed to trajectory frames configured to employ optical and/or EM fiducials for tracking/navigation without requiring MRI imaging.

The navigation systems or devices may be camera-based tracking or navigation systems.

The navigation systems or devices may be EM-based tracking or navigation systems.

Some aspects are directed to trajectory frames configured to hold or employ CT visible image fiducials for non-MRI imaging modalities.

Some aspects are directed to trajectory frames configured to hold or employ optical or EM fiducials for tracking and also configured to include CT visible image fiducials.

Some aspects are directed to navigation systems that can provide information that can be used to confirm that a planned trajectory using an optical or EM tracking/position system is correct to reduce radiation, increase the speed and/or reliability of an intrabody procedure.

The information can include visualizations based (in part) on predefined data of the tool(s) which define a point of interface for the system (*e.*g., software) based on predefined characteristics of the tool(s), *e.g.,* dimensions, shape or configuration and/or known rotational, translational and/or other functional and/or dynamic behavior of one or more surgical tools. The tools can include both imaging modality (e.g., CT) visible fiducials and at least one of (i) EM or (ii) optical tracking markers or fiducials, the latter of which can be detected by a respective EM detector or camera.

The disclosure provides surgical tools or devices with fiducials that can be electronically identified in images generated using one or more imaging modalities such as, for example, CT, X-ray, PET and the like.

In some aspects, the tools/devices with the fiducials can be hybrid tools/devices that can be used for more than one imaging modality, e.g., MRI and CT. The fiducials can comprise a fiducial material that can be CT-visible fluid and an MRI-visible fluid that creates sufficient signal intensity for identifying a location and/or orientation of a target tool.

In some aspects, a trajectory frame can include a base with (i) a plurality of image-modality fiducials arranged so that a line drawn through their centers is a circle, and (ii) a plurality of optic or EM fiducials or tracking members that are concentric with the image-modality fiducials.

Some embodiments of the present invention can provide visualizations to allow more precise control, delivery, and/or feedback of a therapy so that the therapy or a tool associated therewith can be more precisely placed, delivered, confirmed and visualized.

A trajectory frame for use with a surgical system can have a base having a patient access aperture formed therein. The base can be configured to be secured to a body of a patient. The trajectory frame can also include a yoke movably mounted to the base and rotatable about a roll axis, a platform movably mounted to the yoke and rotatable about a pitch axis, and an elongated guide secured to the platform. The guide includes opposite proximal and distal end portions. The guide distal end portion can be positioned proximate the patient access aperture. The guide can include a bore therethrough that extends from the proximal end portion to the distal end portion. The guide can be configured to receive at least one device within the bore. The at least one device can be removably secured to the guide. The at least one device that is received within the bore can be or include an elongate tracking probe mount holding a tracking probe with reflective members arranged in a fixed geometric relationship relative to each other. The reflective members can be configured to be detectable by a camera-based tracking system.

The at least one device can further include a microelectric probe driver adapter. The proximal end portion of the guide can be configured to serially, interchangeably and removably secure the tracking probe mount and the microelectrode probe drive adapter.

The tracking probe can include three spherical reflective members or four spherical reflective members in the fixed geometric relationship relative to each other.

The guide proximal end portion can include at least one longitudinally extending slot that merges into at least one transversely extending slot. The tracking probe mount can be removably secured within the guide bore via lugs extending outwardly therefrom that cooperate with the longitudinally and transversely extending slots to be secured to the guide.

Some aspects are directed to surgical assemblies that include: (i) a trajectory frame holding an elongated guide with a bore and (ii) a plurality of devices configured to be releasably and serially inserted within the bore. The devices include a tracking probe mount holding a tracking probe with an array of reflective members and at least one of the following additional devices: a microelectrode probe driver adapter, a drill guide and drill bit, a skull fixation device and driver, a deep brain stimulation lead guide and a catheter guide. Each device comprises opposite proximal and distal end portions and has outwardly extending lugs on the proximal end portion. Each device can be removably secured to the guide using the lugs that slidably engage the slots.

The trajectory frame can include a base having a patient access aperture formed therein. The base can be configured to be secured to the body of a patient. The trajectory frame can also include a yoke movably mounted to the base and rotatable about a roll axis and a platform movably mounted to the yoke and rotatable about a pitch axis. The elongated guide can be secured to the platform. The guide can have opposite proximal and distal end portions with the guide distal end portion positioned above the patient access aperture. The bore can extend therethrough from the proximal end portion to the distal end portion. The elongated guide can include at least one longitudinally extending slot that merges into at least one transversely extending slot.

The trajectory frame base can be configured to be secured to the scalp or skull of a patient about a burr hole formed therein.

The guide bore can be configured to guide intra-brain placement of at least one device in vivo.

The trajectory frame can include a bracket attached thereto that has an arm that extends outward therefrom and holds a reference frame that has a plurality of spaced apart reflective fiducials in a fixed geometry.

The platform can include an X-Y support table movably mounted to the platform that is configured to move in an X-direction and Y-direction relative to the platform. The guide can be secured to the X-Y support table.

The trajectory frame can include a plurality of spaced apart ears held by and extending laterally outward away from the base, the ears can have upper and lower surfaces and a bracket with a first upright segment residing under and attached to one ear supporting an arm that extends outwardly therefrom. The arm can hold a starburst connector on an end portion of the arm configured to engage a reference frame with an array of optical fiducials thereon for tracking by a camera based tracking system.

The bracket can include a second upright segment residing under and attached to a different ear and a bridging arm extending between the first and second upright brackets.

The bracket starburst connector can have a first swivel axis that allows positional adjustment of an end portion of the arm. The first upright segment can have a starburst connector that has a second swivel axis that is orthogonal to the first swivel axis.

The tracking probe mount can include a collar and stem extending above the collar with a smaller diameter than a body of the tracking probe mount at the collar and extending below the collar. The collar can include an outer wall with at least one aperture extending therethrough or an upwardly extending protrusion with at least one aperture extending therethrough for cooperating with a fixation member that extends through the at least one aperture to lock a lower end portion of the array of the tracking probe to the tracking probe mount.

The base can be configured to be secured to the scalp or skull of a patient about a burr hole formed therein.

The guide bore can be configured to guide intra-brain placement of at least one device in vivo. The trajectory frame can include a bracket attached thereto that has an arm that extends therefrom and holds a reference frame with a plurality of spaced apart reflective members that are configured to be detectable by a camera.

The elongated guide can be secured to the platform and can be configured as a support column that releasably interchangeably holds the plurality of devices.

Some aspects are directed to a trajectory frame for use with a surgical system that includes a base having a patient access aperture formed therein. The base can be configured to be secured to a body of a patient. The trajectory frame can also include a yoke movably mounted to the base and rotatable about a roll axis, a platform movably mounted to the yoke and rotatable about a pitch axis, and an elongated guide secured to the platform. The guide can include opposite proximal and distal end portions. The guide can include a bore therethrough that extends from the proximal end portion to the distal end portion. The guide can be configured to serially and interchangeably receive a plurality of devices within the bore that are removably secured to the guide. One of the devices that is received within the bore can be an elongate tracking probe mount that holds a tracking probe with at least one electromagnetic tracking coil for an electromagnetic surgical navigation system.

The elongate tracking probe mount can include a channel that holds the tracking probe and also comprises outwardly extending lugs that engage longitudinally extending and transversely extending slots of the guide.

Still other aspects are directed to trajectory frames for facilitating surgical navigation. The trajectory frames include a base having a patient access aperture formed therein. The base can be configured to be secured to a body of a patient and has a plurality of ears that reside spaced apart about a perimeter of the base. Trajectory frame can also include a yoke movably mounted to the base and rotatable about a roll axis, a platform movably mounted to the yoke and rotatable about a pitch axis, and an elongated guide secured to the platform. The guide can include opposite proximal and distal end portions with the guide distal end portion positioned above the patient access aperture. The guide can include a bore therethrough that extends from the proximal end portion to the distal end portion. The guide can be configured to receive and removably secure at least one device within the bore. The trajectory frame can include a plurality of toroid-shaped MRI and/or CT visible markers, one on each ear; and a plurality of reflective spherical reflective members. A respective spherical reflective member can reside inside a cavity of a center space of a respective toroid-shaped fiducial marker.

The toroid shaped fiducial markers can be circumferentially spaced apart and arranged about a circle, and wherein lines drawn through centers of the toroid shaped fiducial markers and the reflective spherical members are concentric from a center of the patient access aperture.

Other aspects are directed to a surgical navigation system with a workstation having a User Interface (UI) and a circuit configured to provide a planned trajectory line on an image on a display using a trajectory frame with either or both (a) reflective members detectable by a camera of a camera-based navigation system held by the trajectory frame and/or (b) at least one coil of an (electromagnetic) EM navigation system held by the trajectory frame. The circuit UI is configured to provide a user selectable feature to allow a user to select a second calculation of a planned trajectory line calculated using CT and/or MRI image detectable fiducials held by or on the trajectory frame to confirm the planned trajectory line from the camera or EM navigation system is proper.

The surgical system can be a brain surgery system. The circuit can have predefined data of physical characteristics of the trajectory frame with the MRI and/or CT image fiducial markers including a plurality of image fiducial markers that surround a base opening over a patient access aperture.

The circuit can be configured to provide the planned trajectory line on a patient image and/or rendered image using patient image data on the display using the reflective members detectable by the camera of the camera-based navigation system.

The circuit can be configured to segment image data to locate the image fiducials for the user selectable second calculation. The image fiducials can be circumferentially spaced apart about the patient access aperture and positioned relative to each other such that at least two are closer together than another to define an affirmative orientation of the trajectory frame.

The image fiducials can include at least three fluid-filled annular MRI and/or CT detectable image fiducials. The reflective members can include at least three reflective members on the trajectory frame detectable by the camera of the camera-based navigation system.

The fiducial markers can include MRI and/or CT detectable image fiducial markers and reflective members detectable by a camera based tracking system and held within a line of sight of a camera of a camera-based navigation system.

The elongate tracking probe mount can include a channel that holds the tracking probe and also comprises outwardly extending lugs that engage longitudinally extending and transversely extending slots of the guide.

The tracking probe mount and the trajectory frame can include image fiducials detectable in CT and/or MRI images to thereby allow a circuit to calculate an intrabody trajectory in two different methods, one calculated using image interrogation of images with the image fiducials and another calculated using a camera-based system with the reflective members.

The tracking probe mount can include a longitudinally extending through channel. The tracking probe mount and the trajectory frame can include image fiducials detectable in CT and/or MRI images.

The tracking probe mount and the trajectory frame can include image fiducials detectable in CT and/or MRI images to thereby allow a circuit to calculate an intrabody trajectory in two different methods, one calculated using image interrogation of images with the image fiducials and another calculated using an EM surgical navigation system using the tracking probe EM tracking coil.

### BRIEF DESCRIPTION OF THE DRAWINGS

**Fig. 1** is a schematic illustration of an image-guided surgical system
**Fig. 2** is a schematic illustration of an image-guided surgical system with cameras
**Fig. 3** is a schematic illustration of an MRI-guided surgical system.
**Fig. 4** is a side view of a conventional fiducial marker arrangement for a neurosurgery.
**Fig. 5** is a series of actions that can be used during an image-guided surgery
**Fig. 6** is a schematic illustration of an exemplary screen shot of a User Interface
**Fig. 7** is a prophetic screen shot of an exemplary display of a workflow group associated with a User Interface provided to a user to facilitate an image-guided procedure.
**Figs. 8A-8C** are schematic illustrations of target scan planes that can be above a patient's head to identify a trajectory or grid entry while reducing or minimizing radiation exposure to a patient.
**Fig. 9A** is a schematic of an exemplary trajectory frame.
**Fig. 9B** is a side view of the trajectory frame shown in **Fig. 9A** illustrating a depth stop with a cooperating elongate member.
**Fig. 9C** is a section view of the trajectory frame taken along line **9C-9C** in **Fig. 9A** illustrating a targeting cannula.
**Fig. 10** is a top view of a base of a trajectory frame with fiducials.
**Fig. 11A** is a side perspective view of the base shown in **Fig. 10****.**
**Fig. 11B** is a schematic side view of a portion of the trajectory frame with a post for holding a spherical optical tracking member.
**Fig. 12** is a schematic illustration of a marking grid patch and associated screen display of coordinates of a surgical entry site.
**Figs. 13A-13E** are schematic illustrations of grid segmentation and grid deformation that can be used to define an entry site location.
**Figs. 14A-14B** are schematic illustrations of a base or frame marker segmentation that can be used to define position and orientation of a base or frame of a trajectory frame using both optical or EM position/tracking and image segmentation of different but concentrically aligned fiducial markers.
**Fig. 15** is a screen shot of an exemplary prophetic workflow group and/or steps associated with a User Interface provided to a user to facilitate an image-guided procedure.
**Fig. 16** is a data processing system.
**Fig. 17** is a side perspective view of a trajectory frame with an optical tracking probe according to embodiments of the present invention.
**Fig. 18A** is a side perspective view of a trajectory frame with both an optical tracking probe and an optical reference frame attached to the trajectory frame according to embodiments of the present invention.
**Fig. 18B** is a side perspective view of a trajectory frame with an optical tracking probe with a through channel with image fiducials (e.g., fluid-filled segments detectable in MRI and/or CT images) and an optional optical reference frame attached to the trajectory frame according to embodiments of the present invention.
**Fig. 19A** is a side perspective view of a tracking probe mount holding the tracking probe for releasable attachment to the support column of the trajectory frame shown in **Figs. 17** and **18A****.**
**Figs. 19B** and **19C** are side perspective views of exemplary tracking probe mounts, shown without the tracking probe.
**Fig. 19D** is a side perspective view of a tracking probe mount holding the tracking probe for releasable attachment to the support column of the trajectory frame shown in **Fig. 18B**.
**Figs. 19E** and **19F** are side perspective views of exemplary tracking probe mounts, shown without the tracking probe.
**Fig. 19G** is a section view of an exemplary tracking probe mount shown in **Figs. 19E/19F**.
**Fig. 20** is a side perspective view of the trajectory frame shown in **Fig. 18A****,** but shown without the tracking probe.
**Fig. 21A** is an enlarged side perspective view of a bottom portion of the trajectory frame shown in **Fig. 17** illustrating exemplary attachment configurations.
**Fig. 21B** is a greatly enlarged partial bottom perspective view of the bracket shown in **Fig. 21A****.**
**Fig. 22A** is a top perspective view of an exemplary bracket for attaching a reference frame to the trajectory frame.
**Fig. 22B** is an assembled view of the bracket shown in **Fig. 22A** to attachment segments of the trajectory frame.
**Fig. 23A** is a side perspective view of another exemplary attachment bracket.
**Fig. 23B** is an enlarged partial assembly view of a portion of a connector attached to the reference frame bracket shown in **Fig. 23A****,** assembled to an attachment segment of the trajectory frame.
**Fig. 23C** is an enlarged partial assembly view of the reference frame attachment bracket shown in **Fig. 23A**.
**Fig. 24** is a side perspective view of the trajectory frame shown in **Figs. 17** and **18A** illustrating the support column releasably holding a microelectric (MER) probe drive adapter, typically for awake deep brain surgeries.
**Fig. 25A** is a side perspective view of the MER probe drive adapter shown in **Fig. 24** .
**Fig. 25B** is a partial exploded view of the MER probe drive adapter and trajectory frame shown in **Fig. 25A**.
**Fig. 26** is a side perspective view of a trajectory frame holding a probe driver using the MER probe driver adapter shown in **Figs. 28****,** **29A,** and 29B.
**Fig. 27A** is a side perspective view of a trajectory frame holding a universal tracker.
**Fig. 27B** is a side/front view of the assembly shown in **Fig. 27A****.**
**Fig. 27C** is a front view of a tracker guide holding the universal tracker for releasable attachment to a support column/guide of the trajectory frame.
**Fig. 27D** is a front view of the tracker guide without the universal tracker shown in **Figs. 27A-C** .
**Fig. 28** is a side view of a trajectory frame illustrating the tracking probe shown in **Figs. 27A-27D** replaced by a device (DBS lead) guide.
**Fig. 29** is a schematic illustration of a camera-based navigation system.
**Fig. 30A** is a schematic illustration of a trajectory frame with an EM tracking probe.
**Fig. 30B** is a schematic illustration of a tracking probe with a cooperating mount to attach to the trajectory frame shown in **Fig. 30A****.**
**Fig. 31** is a schematic illustration of an EM-based navigation system.

### DETAILED DESCRIPTION OF EMBODIMENTS OF THE INVENTION

The present invention will now be described more.

In the drawings, the thickness of lines, layers, features, components and/or regions may be exaggerated for clarity and broken lines (such as those shown in circuit of flow diagrams) illustrate optional features or operations, unless specified otherwise. In addition, the sequence of operations (or steps) is not limited to the order presented in the claims unless specifically indicated otherwise.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprises" and/or "comprising," when used in this specification, specify the presence of stated features, steps, operations, elements, and/or components, but do not preclude the presence or addition of one or more other features, steps, operations, elements, components, and/or groups thereof. As used herein, the term "and/or" includes any and all combinations of one or more of the associated listed items.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the specification and relevant art and should not be interpreted in an idealized or overly formal sense unless expressly so defined herein. Well-known functions or constructions may not be described in detail for brevity and/or clarity.

It will be understood that when a feature, such as a layer, region or substrate, is referred to as being "on" another feature or element, it can be directly on the other element or intervening elements may also be present. In contrast, when an element is referred to as being "directly on" another feature or element, there are no intervening elements present. It will also be understood that, when a feature or element is referred to as being "connected" or "coupled" to another feature or element, it can be directly connected to the other element or intervening elements may be present. In contrast, when a feature or element is referred to as being "directly connected" or "directly coupled" to another element, there are no intervening elements present. Although described or shown with respect to one embodiment, the features so described or shown can apply to other embodiments.

Visualizations can be shown on a screen or display so that the map and/or anatomical or tool structure is in a flat 2-D view and/or in 2-D what appears to be 3-D volumetric images with data representing features or electrical output with different visual characteristics such as with differing intensity, opacity, color, texture and the like. A 4-D map illustrates time-dependent activity, such as electrical activity or blood flow movement.

The systems can be configured to operate based on known physical characteristics of one or more surgical tools such that the hardware is a point of interface for the circuit or software. The systems can communicate with databases that define dimensions, configurations or shapes and spacing of components on the tool(s). The defined physical data can be obtained from a CAD model of a tool and/or a geometric shape of an array of defined fiducials associated therewith. The physical characteristics can include dimensions or other physical features or attributes and may also include relative changes in position of certain components or features upon a change in position of a tool or portion thereof. The defined physical characteristics can be electronically (programmatically) accessible by the system or known *a priori* and electronically stored locally or remotely and used to automatically calculate certain information and/or to segment image data. That is, the tool data from the model can be used to segment image data and/or correlate a position and orientation of a tool and/or provide trajectory adjustment guidelines or error estimates, warnings of improper trajectories and the like. For example, a grid for marking a burr hole location and/or a trajectory frame that adjusts an intrabrain path for placing a diagnostic or therapy device and such can be input, transposed, and/or overlaid in a visualization of the tool and patient structure or otherwise used, such as, for example, to project the information onto a patient's anatomical structure or determine certain operational parameters including which image volume. At least some of the resulting visualizations are not merely an MRI or CT image of the patient during a procedure.

The visualizations can be rendered visualizations that can combine multiple sources of data to provide visualizations of spatially encoded tool position and orientation with anatomical structure.

The systems, methods, circuits, devices, computer programs and the like can be used to generate two different trajectories or grid entry points, one calculated from conventional optic or EM systems and a supplemental or additional trajectory or grid entry coordinates using the CLEARPOINT® navigation system and/or SMARTFRAME® trajectory frame from MRI Interventions, Memphis, TN, modified for non-MRI imaging modalities, e.g., camera tracking systems which may include images from any imaging modality e.g., CT, X-ray, PET and the like, which may be imported and registered to a patient orientation for surgery. In the CLEARPOINT® surgical systems, the image space is the surgical space thus not requiring separate registration.

The term "ACPC coordinate space" refers to a right-handed coordinate system defined by anterior and posterior commissures (AC, PC) and Mid-Sagittal plane points, with positive directions corresponding to a patient's anatomical Right, Anterior and Head directions with origin at the mid-commissure point.

The term "grid" refers to a pattern of crossed lines or shapes used as a reference for locating points or small spaces, *e.g.,* a series of rows and intersecting columns, such as horizontal rows and vertical columns (but orientations other than vertical and horizontal can also be used). The grid can include associated visual indicia such as alphabetical markings (*e.g*., A-Z and the like) for rows and numbers for columns (*e.g*., 1-10) or the reverse. Other marking indicia may also be used. The grid can be provided as a flexible patch that can be releasably attached to the skull of a patient. For additional description of suitable grid devices, *see* U.S. Patent Application Publication US2009/0177077.

The term "fiducial marker" is used interchangeably with the term "fiducial" and refers to a marker that can be electronically identified using a detector, image recognition and/or electronic interrogation of image data for tracking position of a device in a surgical environment. The fiducial marker can be provided in any suitable manner, such as, but not limited to, a geometric shape of a portion of the tool, one or more components held by, on or in the tool, an electromagnetic tracking member (e.g., sensor or detector having a coil or coil array), a coating or fluid-filled component or feature (or combinations of different types of fiducial markers) that makes the fiducial marker(s) visible to a camera and/or in image data of a target imaging modality so that the image data has sufficient signal intensity (brightness) for identifying location and/or orientation information for the tool and/or components thereof in space. The fiducial markers or tracking members can include electromagnetic markers/members or passive optical markers, for example.

The term "visible" with respect to an image means that the device, feature or component, is visible, directly or indirectly, in an image. The visibility may be indicated by the increased SNR of the signal proximate the fiducial marker relative to an adjacent location without such fiducial marker.

The term "MRI compatible" means that the so-called component(s) is safe for use in an MRI environment and as such is typically made of a non-ferromagnetic MRI compatible material(s) suitable to reside and/or operate in a high magnetic field environment. The term "high-magnetic field" refers to field strengths above about 0.5 T, typically above 1.0T, and more typically between about 1.5T and 10T. MRI Scanners are well known and include high-field closed bore and open bore systems.

Aspects of the disclosure can be configured to carry out diagnostic and interventional procedures such as to guide and/or place interventional devices to any desired internal region of the body or object, but may be particularly suitable for neurosurgeries. The object can be any object, and may be particularly suitable for animal and/or human subjects. Although primarily described with respect to placement of stimulation leads in the brain, the invention is not limited thereto. For example, the system can be used for gene and/or stem-cell based therapy delivery or other neural therapy delivery and allow user-defined custom targets in the brain or to other locations. In addition, aspects of the systems can be used to ablate tissue in the brain or at other locations.

Examples of known treatments and/or target body regions are described in U.S. Patent Nos. 6,708,064; 6,438,423; 6,356,786; 6,526,318; 6,405,079; 6,167,311; 6,539,263; 6,609,030 and 6,050,992.

Aspects of the disclosure may take the form of an entirely software embodiment or an embodiment combining software and hardware aspects, all generally referred to herein as a "circuit" or "module." In some aspects, the circuits include both software and hardware and the software is configured to work with specific hardware with known physical attributes and/or configurations. Furthermore, aspects of the disclosure may take the form of a computer program product on a computer-usable storage medium having computer-usable program code embodied in the medium. Any suitable computer readable medium may be utilized, including hard disks, CD-ROMs, optical storage devices, a transmission media such as those supporting the Internet or an intranet, or other storage devices.

Computer program code for carrying out operations of the present disclosure may be written in an object oriented programming language such as Java®, Smalltalk or C++. However, the computer program code for carrying out operations of the present disclosure may also be written in conventional procedural programming languages, such as the "C" programming language. The program code may execute entirely on the user's computer, partly on the user's computer, as a stand-alone software package, partly on the user's computer and partly on another computer, local and/or remote or entirely on the other local or remote computer. In the latter scenario, the other local or remote computer may be connected to the user's computer through a local area network (LAN) or a wide area network (WAN), or the connection may be made to an external computer (for example, through the Internet using an Internet Service Provider).

The present invention is described in part below with reference to flowchart illustrations and/or block diagrams of methods, apparatus (systems) and computer program products according to embodiments of the disclosure It will be understood that each block of the flowchart illustrations and/or block diagrams, and combinations of blocks in the flowchart illustrations and/or block diagrams, can be implemented by computer program instructions. These computer program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer-readable memory that can direct a computer or other programmable data processing apparatus to function in a particular manner, such that the instructions stored in the computer-readable memory produce an article of manufacture including instruction means which implement the function/act specified in the flowchart and/or block diagram block or blocks.

The computer program instructions may also be loaded onto a computer or other programmable data processing apparatus to cause a series of operational steps to be performed on the computer or other programmable apparatus to produce a computer implemented process such that the instructions which execute on the computer or other programmable apparatus provide steps for implementing the functions/acts specified in the flowchart and/or block diagram block or blocks.

The flowcharts and block diagrams of certain of the figures herein illustrate exemplary architecture, functionality, and operation of possible implementations of embodiments of the present disclosure In this regard, each block in the flow charts or block diagrams represents a module, segment, or portion of code, which comprises one or more executable instructions for implementing the specified logical function(s). It should also be noted that in some alternative implementations, the functions noted in the blocks may occur out of the order noted in the figures. For example, two blocks shown in succession may in fact be executed substantially concurrently or the blocks may sometimes be executed in the reverse order or two or more blocks may be combined, depending upon the functionality involved.

Generally stated, embodiments of the systems are configured to provide a substantially automated or semi-automated and relatively easy-to-use navigation and/or image-guided systems with defined workflow steps and interactive visualizations. In particular embodiments, the systems can define and present workflow with discrete steps for finding target and entry point(s), localizing the entry point(s) to a physical identified grid position, guiding the alignment of the trajectory frame with a guide to a planned trajectory, monitoring the insertion of a surgical tool, such as a probe, needle, microelectrodes, leads and the like.

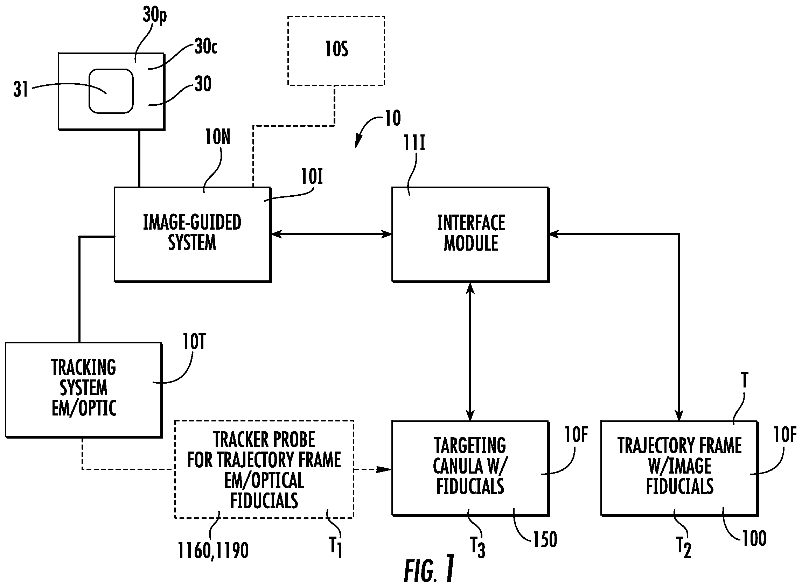

The systems can be configured to electronically confirm or compare for concordance, a projected trajectory generated using image fiducials (image fiducials generate small regions of increased SNR in CT and/or MRI image data) on a trajectory frame and/or held by the trajectory frame compared to EM or optical markers/fiducials on the trajectory frame or held by the trajectory frame. The trajectory calculated using the fiducials can be used to provide scan coordinates to the CT or other image scanner to narrowly select the slab of interest to reduce radiation exposure. The workstation/circuit **30c** can passively or actively communicate with an image (e.g., CT or MRI) scanner. The circuit or computer module interface **111 (****Fig. 1****)** can select, and/or display data on a display **31** for, a scan plane, which can be entered at the workstation **30.**

Aspects will now be described in further detail below with reference to the figures. **Fig. 1** illustrates a surgical system **10** with a navigation system **10N** that can optionally communicate with a CT and/or MRI scanner **10S** and a clinician workstation **30** with at least one circuit **30c** and at least one display **31.** The navigation system **10N** also includes a tracking system **10T** such as an EM or optic tracking system with a camera and an interface module **111** that can communicate with the tracking system **10T** and the navigation system **10.** The term "image guided system" is used generally to refer to surgical navigation systems that include patient images (which may be acquired before a surgery and/or at defined points during a surgery to confirm location) but does not require a continuous series of images during the surgery.

The tracking system **10T** and/or interface module **111** can be onboard or remote from the workstation **30,** the scanner **10S** or the navigation system **10N.** Examples of imaging system modalities include, among other things, magnetic resonance imaging (MRI), computed tomography (CT), positron emission tomography (PET), and single photon emission computed tomography (SPECT) and fluoroscopic systems. In certain embodiments, the imaging modality is a radiation-based imaging modality such as CT. In some aspects, the system **10** includes ultrasound and/or X-ray.

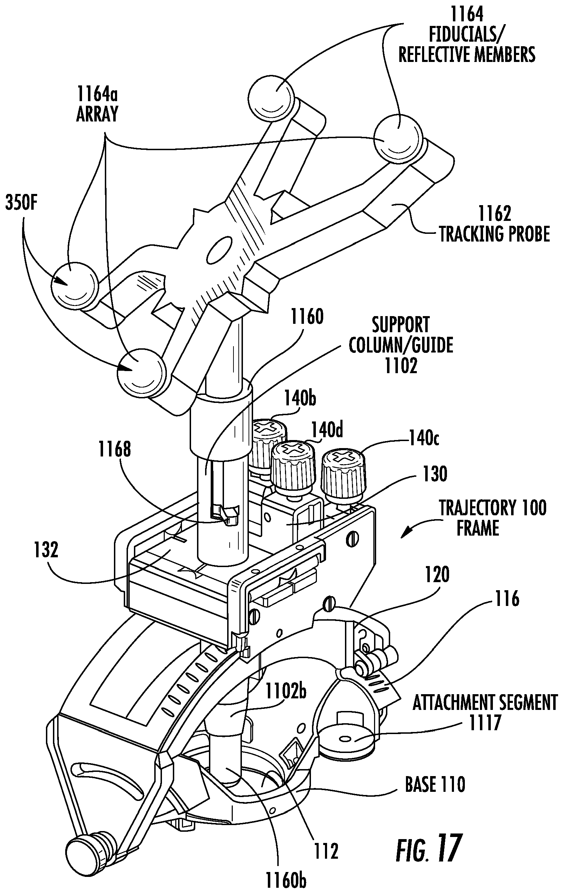

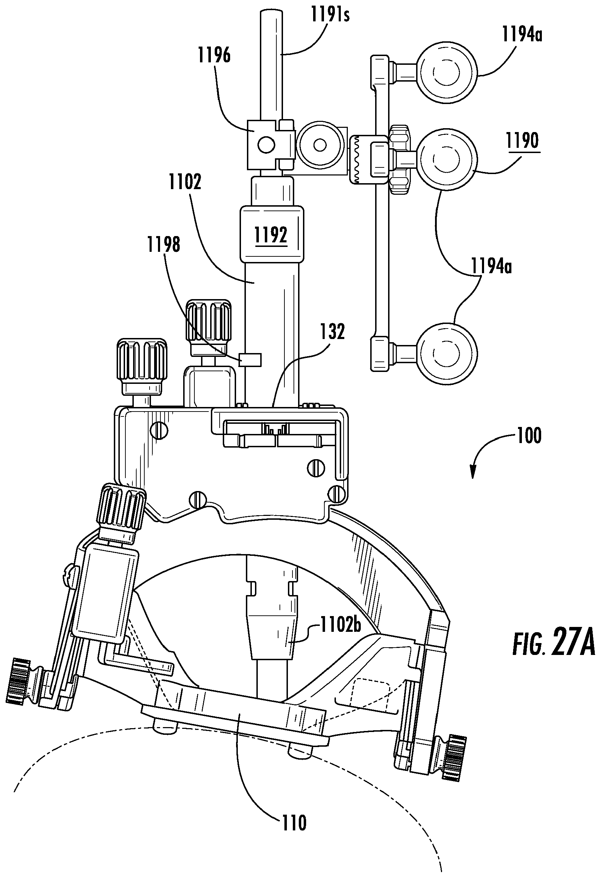

The interface module **111** can be configured to have an image evaluation protocol for defined tools **T** such as that used by the CLEARPOINT® surgical system for the SMARTFRAME® trajectory frame and/or SMARTGRID® surgical patch, all from MRI Interventions, Inc., Memphis, TN. The interface module **111** can track optical fiducials and/or EM fiducials and/or interrogate images for orientation and location of the tools **T** on a patient and calculate an intrabody trajectory and/or grid entry coordinates using fiducials. The system **10** can include and at least one interventional and/or surgical tool **"T"** shown as a plurality of tools **T₁-T₃,** including a tracker probe **1160, 1190** (*see also,* **Figs. 17****,** **27A**), a targeting cannula **150** (this tool is typically only required for MRI uses but may be useful for some systems that use input from images from a CT imaging modality), and a trajectory frame **100.** Further details of a surgical system, targeting cannula, trajectory frame and grid can be found in one or more of U.S. Patent Nos. 8,340,743; 8,195,272; 8,175,677; and 8,315,689.

A scanner interface **101** may be used to allow communication between the workstation **30,** the navigation system **10N** (e.g., tracking system **10T** and/or tracking interface module **111**) and/or the (CT and/or MRI) scanner **10S.** The system interface **101** and/or circuit **30c** may be hardware, software or a combination of same. The interface **101** and/or circuit **30c** may reside partially or totally in the scanner **10S,** partially or totally in the workstation **30,** or partially or totally in a discrete device or devices (e.g., processors) or server(s).

The surgical systems **10** can both track one or more tools **T,** serially or concurrently, using the tracking system **10T** as in conventional image guided systems. The surgical systems **10** can be configured to allow an automated or user-selected supplemental or confirmatory action using the tracking interface module **111** to segment image data and place the tool **T** in the rendered visualization in the correct orientation and position in 3D space, anatomically registered to a patient. The tools **T** can include tracking or position determination components such as (EM and/or optic) fiducials **10F.**

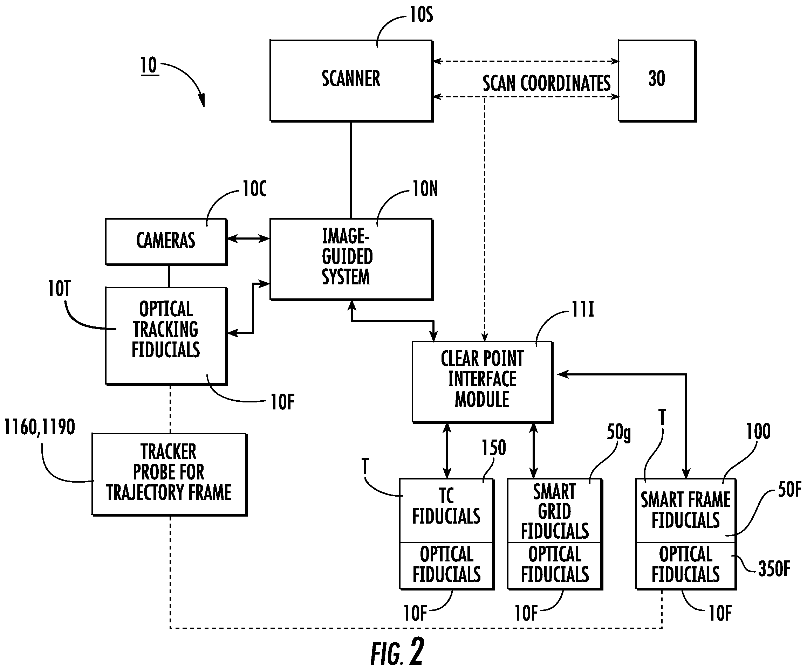

**Fig. 2** illustrates that the system **10** can include an optic tracking system **10T** with optical tracking fiducials **10F** that may reside both on the patient and/or on one or more of the tools **T.** The system **10** can include one or more tracking cameras such as CCD cameras **10C** for tracking fiducials **10F** such as optical fiducials (e.g., reflective members) for visual feedback to a display **30,** such as with respect to target anatomy **32.** Such cameras are well known to those of skill in the art. Fiducial markers that can be located and recognized by an imaging system or other system are useful in neurosurgery and other applications. *See, e.g.,* U.S. Patent No. 8,073,530.

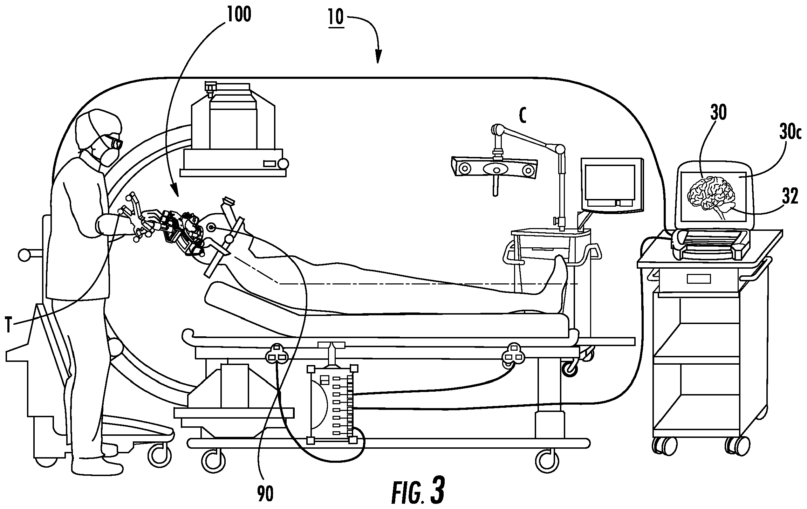

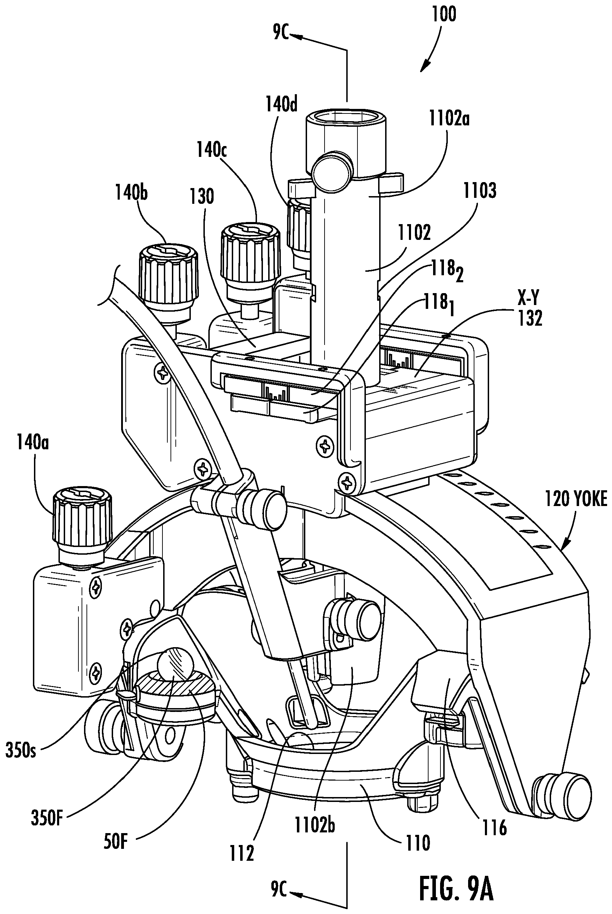

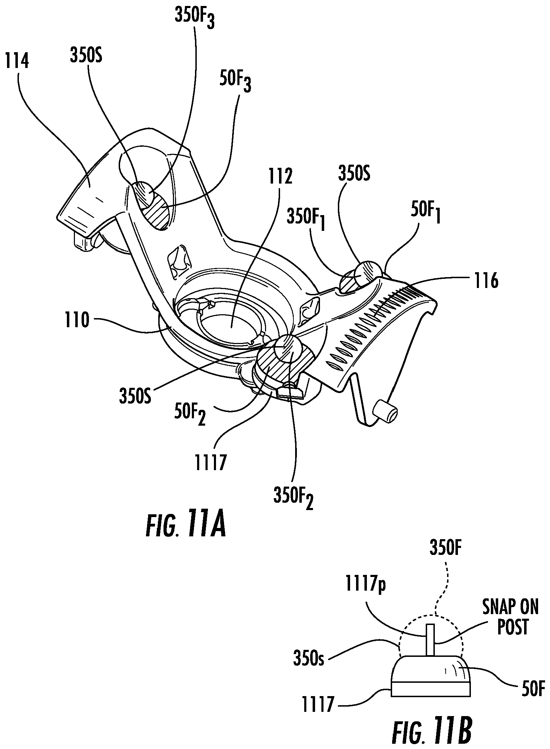

**Fig. 3** is a schematic of an image guided system **10** that employs a navigation system which communicates with an imaging system **10S.** The imaging system **10S** can comprise or communicate with a CT or MRI Scanner, for example. Typical operation of an exemplary system is described in U.S. Patent No. 8,150,494. As shown, the system **10** includes at least one trajectory frame **100** that can include fiducials **10F** with both image fiducials **50F,** e.g., fluid-filled toroid shaped components **(****Fig. 11****)** and optical fiducials **350F,** e.g., reflective members (**Figs. 9A-9C****,** **10** and **11**). The MRI and/or CT visible image fiducials **50F** can also or alternatively include those associated with a targeting cannula **150** held in the trajectory frame **100,** e.g., a fluid filled longitudinally extending channel merging into a sphere bounding a lower end of a through-channel.

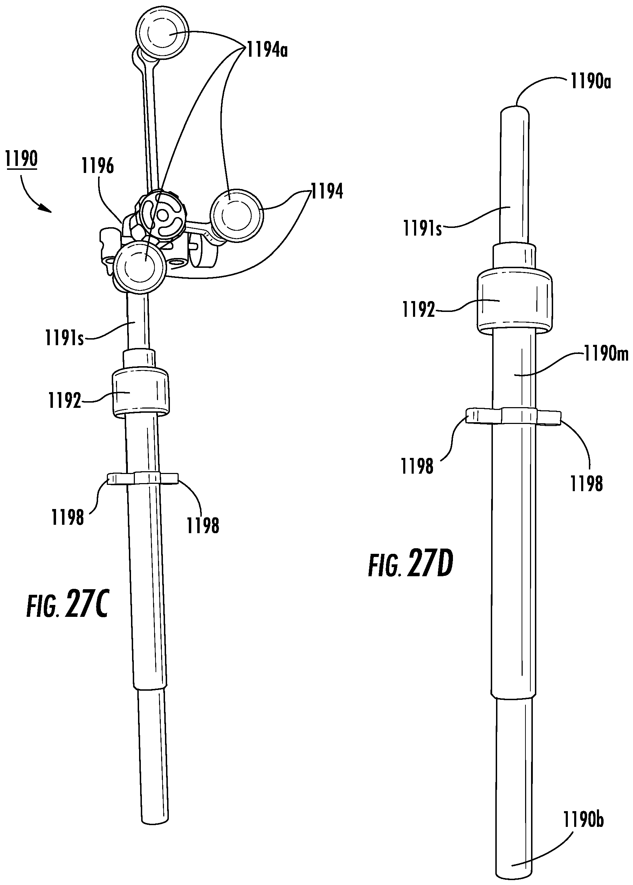

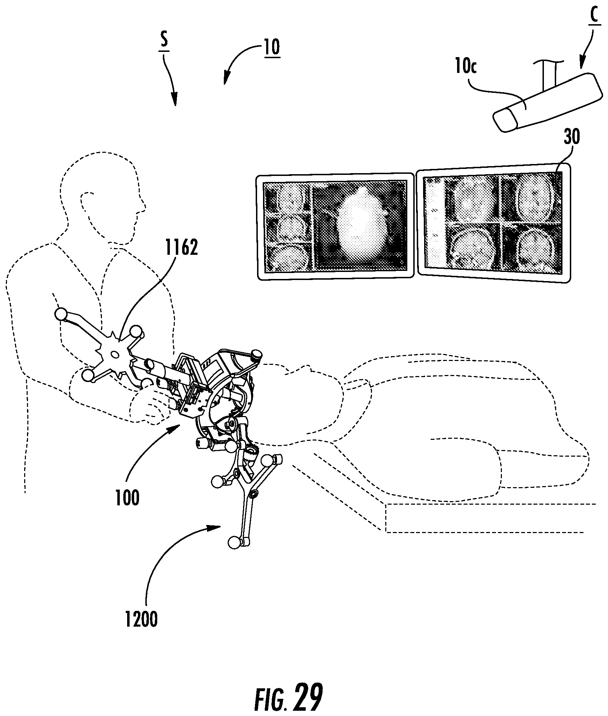

In some embodiments, the system **10** is an optically-tracked system using a camera **C/10C** of a tracking or navigation system **10N (****Figs. 2****,** **29****)** that detects optical fiducials **350F,** e.g., reflective patches or components such as balls or spheres (which can include some on a patient for registration, **Fig. 3**) and/or on tools and/or tracker probes **T** for tracking (*see, e.g.,* **Figs. 17-29**). The systems **10** can be configured to calculate locations of the reflective members (such as, for example, tape, patches or balls/spheres) and/or to detect a defined geometric pattern of the array of fiducials **1164a, 1194a (****Figs. 17****,** **27C****),** for example. These systems **10** can be modified as described herein to have a co-registration module or a concordance review using a different trajectory calculation module that does not require registration as described herein.

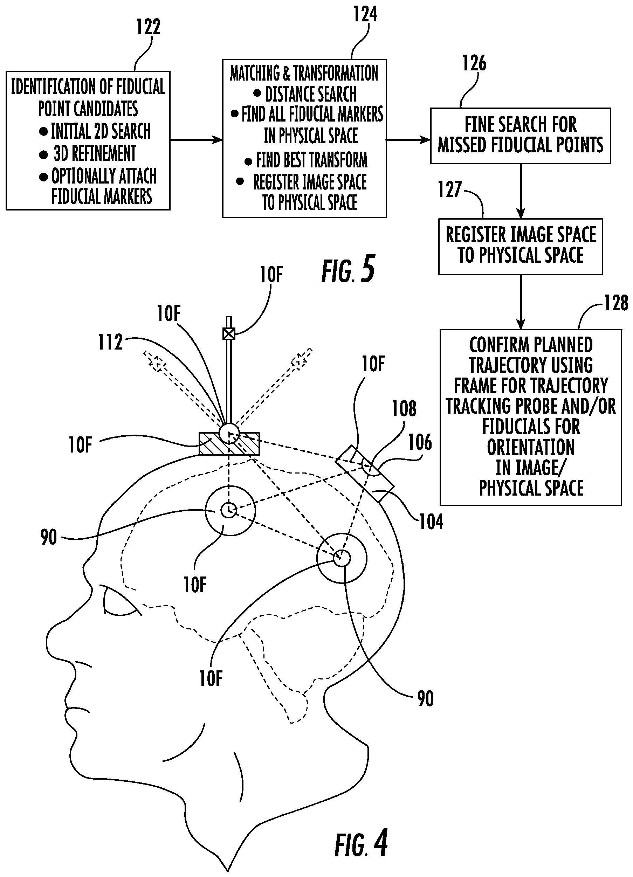

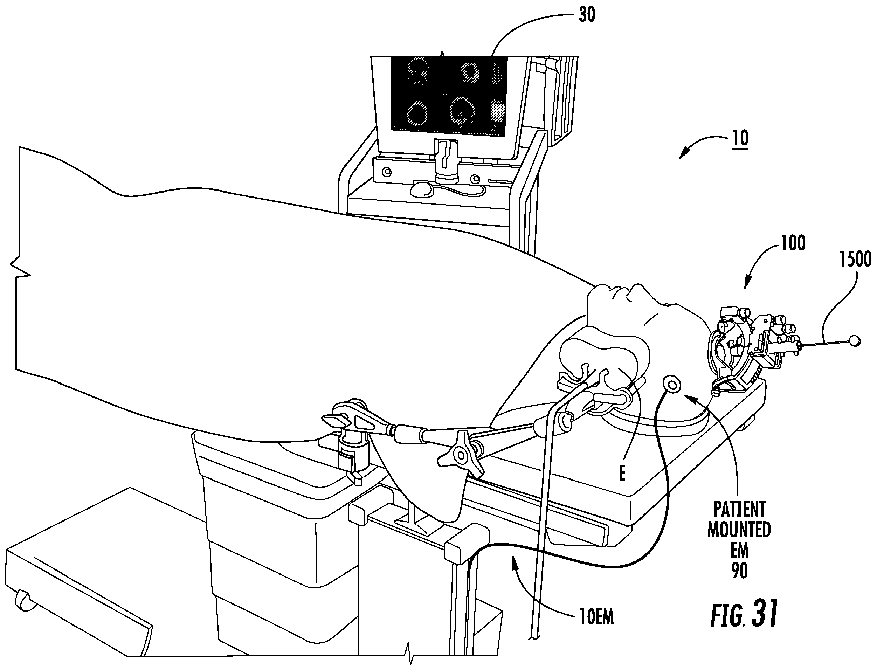

**Fig. 4** is a schematic illustration of optical fiducials **10F.** Further description is provided in U.S. Patent No. 8,150,494. Steps **122, 124, 126, 127** and **128** can be carried out as shown in **Fig. 5** to facilitate image-guided surgeries. However, other steps or systems can be used for registration. The trajectory frame **100** can be placed on the head of the patient before the registration and a tracker probe **1160, 1190** can be used to define a desired intrabody trajectory **(****Figs. 17****,** **27A****).** It is also contemplated that while images may be obtained during surgery, continuous or ongoing imaging with an imaging modality such as CT is not required. Rather, one or more pre-operative images (e.g., an MRI image) can be used (often registered to a CT day of surgery image) along with a visualized trajectory line from a tracking system such as a camera with optical fiducials or an EM tracking system to provide navigation input to the pre-op image(s) presented on a display (e.g., **Fig. 29****,** **31**).

In some embodiments, such as for neuro/brain surgeries, the planned intrabody trajectory, with the trajectory frame **100** on the patient, can be calculated and/or confirmed in an MRI scanner, post-skull opening. That is, the planned trajectory from the non-MRI image guided system can be compared to a trajectory calculated using MRI image data after the skull is opened. The remainder of the surgical procedure can be carried out in an OR (operating room) with a camera based system, with the optical fiducials used to register the patient in the surgical space. This embodiment may reduce the time demand on MRI scanner systems, but suitable sterility conditions should be observed when changing patient venues.

The system **10** can be configured to provide workflow for a unilateral or bilateral (or even a trilateral or more) procedure. Selection of the procedure type can initiate an associated work flow progression that is presented on a display with associated patient image views.

As shown in **Figs. 3** and **4****,** the system **10** can optionally include patient-mounted fiducial markers **10F** shown as features **90** that can be any appropriate marker to be interconnected with the patient. For example, the makers sold by IZI Medical Products, Baltimore, Md. can be used. The markers **90** can be for patient registration and can include an adhesive base that can be adhered to the dermis of the cranium. The fiducial markers can be associated or connected to the patient with an adhesive, mounting screw, clamp, etc. According to various aspects, the fiducial markers **90** can be attached to the patient with an adhesive prior to acquiring image data in a selected manner. The fiducial markers **90** can be placed in predetermined locations or in random locations for imaging. The fiducial markers **90** can also include a body or cavity **104** extending from the adhesive base. The body or cavity **104** can include a material that can be imaged in a selected imaging modality. For example, the cavity **104** can include a material that can be imaged in a CT, MRI, a PET scan, or any appropriate scan or combination of scans. Therefore, the fiducial marker **90** can be used in one or a plurality of imaging modalities to acquire image data of the patient. The fiducial marker **90** can also include a fiducial divot or identification divot **106.** The divot **106** can be provided as a hemispherical or portion of the hemisphere. The divot **106** can, therefore, define a center point **108** that can be within or outside of the divot **106.** The center point **108** can be used by the navigation system **10N** to identify a point relative to the patient for the registration of the image data to the patient space, as discussed further herein. The center point **108** can also be referred to as a fiducial marker, in general for registration. Also, as discussed herein, the center point **108** can be the region of the fiducial marker that is identified in the image data as the fiducial point. Thus, registration can occur between the fiducial points in the image data and the fiducial markers **90** by finding the center point **108** of the fiducial markers **90.**

The navigation system **10N** may also perform registration using anatomic surface information or path information as is known in the art (and may be referred to as auto-registration). The system **10** may also perform 2D to 3D registration by utilizing the acquired 2D images to register 3D volume images by use of contour algorithms, point algorithms or density comparison algorithms, as is known in the art. An exemplary 2D to 3D registration procedure is set forth in U.S. Pat. App. Pub. No. 2004/0215071, entitled "Method and Apparatus for Performing 2D to 3D Registration" filed on Aug. 20, 2003.

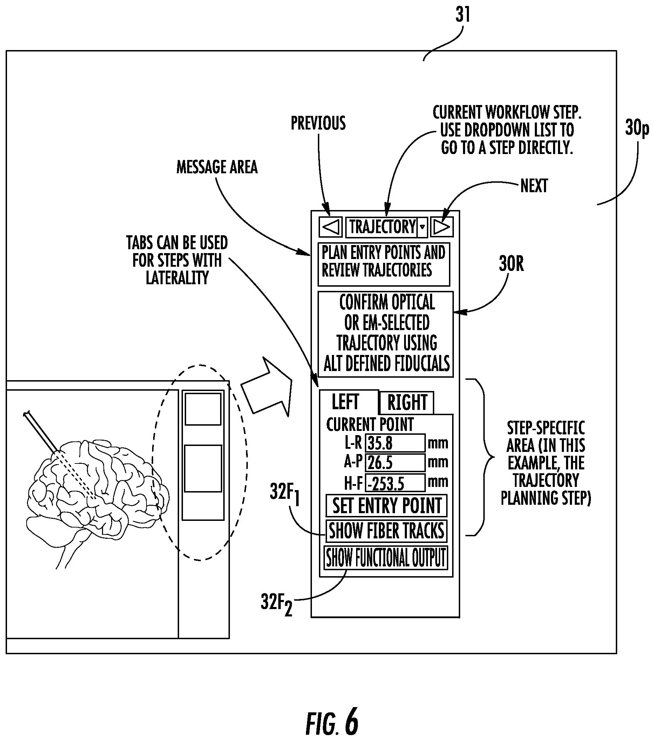

**Fig. 6** illustrates an example of a workstation window or panel **30p** on display screen **31.** The panel **30p** can illustrate a current workflow step and allow a user to go to a step directly (such as via a drop down list or selection of a workflow step in a toolbar or the like) and can be presented adjacent different views of the intrabody trajectory and patient anatomy. Tabs or other user-selectable features, panels or windows, with visual feedback on status of a step for each side can be used for steps with laterality (*e.g.,* left or right for bilateral procedures).

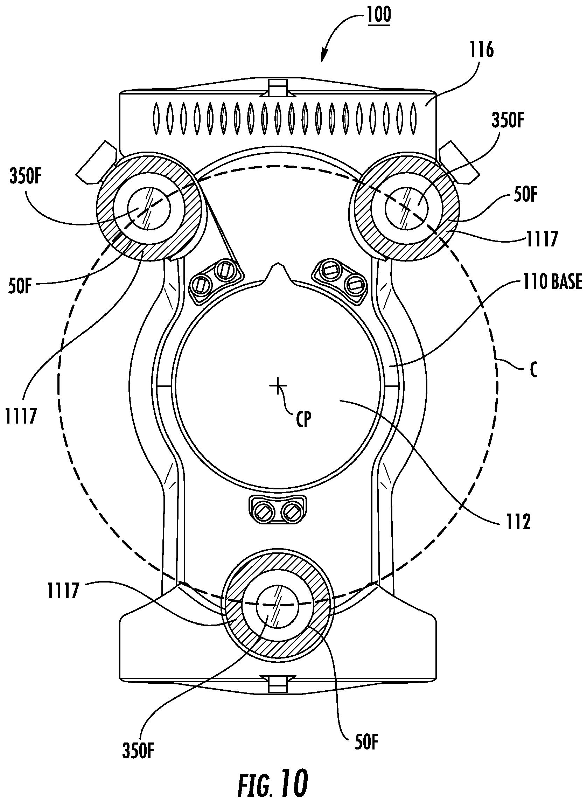

One option in a workflow can include a "Confirm" optical or EM calculated trajectory (and/or grid entry) **30R** using a different (alternate and defined) type of fiducial from the optical fiducials used by the camera **10C (****Fig. 29****)** based tracking system **10N.** The actual descriptor for this action can vary but this can allow a user or the system to electronically calculate the trajectory using an identified location and/or position of at least one image fiducial, such as image fiducial **50F** on a base **110** of the trajectory frame **100 (****Figs. 9A-9C****,** **10****)** around the patient access aperture **112.** The display **31** can include viewer tools such as zoom, pan, width/level, magnifier, etc.

The scanner **10S** can include a console that has a "launch" application or portal for allowing communication to the circuit **30c** of the workstation **30.** In some embodiments, a dynamic or active communication protocol between two or more of the circuit **30c,** workstation **30** and the Scanner **10S** may be used to acquire tracking data, image data and/or initiate or request a particular targeted scan volume.

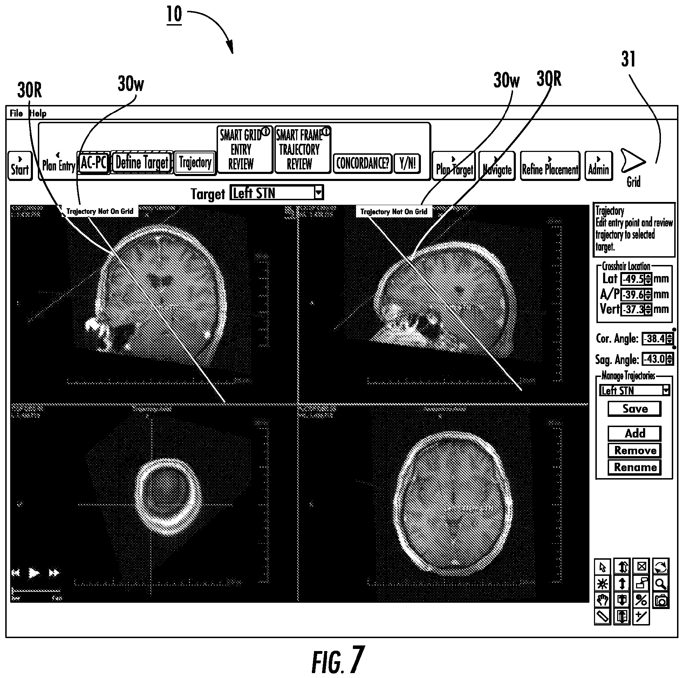

As shown in **Fig. 7****,** generally described, for some unilateral scenarios, the user will proceed through a set of discrete workflow steps to load patient image data, identify a target point, identify an entry point and/or a planned trajectory, and align a tool in the trajectory frame **100.** Optionally, the system **10** can also allow a user to select a "confirm" trajectory and/or grid entry review to assess concordance "Y" or "N" with a camera-based conventionally calculated "identified" trajectory and/or grid entry.

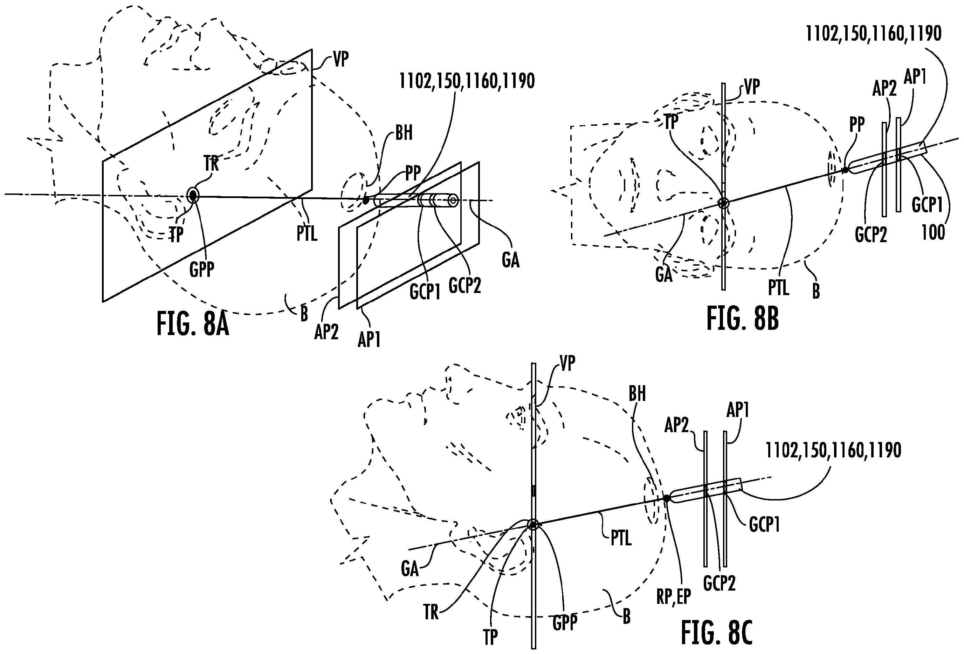

**Figs. 8A-8C** illustrate that the image scan volumes can be minimized by using scan planes above a patient's head that intersect (perpendicular to) a long axis of a guide or support column **1102 (****Figs. 9A****,** **17****)** of the trajectory frame **100,** a tracker probe **1160** or **1190** or **1500 (****Figs. 17****,** **27A****, 33A)** in the trajectory frame **100,** or a targeting cannula **150** in the trajectory frame **100.** For example, the system **10** can optionally prescribe a thin scan plane for the target point **TP.** Alternatively or additionally, the system **10** can prescribe a scan segment that is above the patient's head as a thin slab of about 4 cm by about 2 cm inclusive of a portion of the tracking probe **1160** or **1190** or **1500** or the targeting cannula **150.** A discussion of a similar protocol used for MRI scan selection and trajectory guidance is described in U.S. Patent No. 8,340,743. Generally stated, in some embodiments, a planned trajectory line **PTL** may be determined by defining a line extending through the target point **TP** and a selected reference point **RP.** According to some embodiments, the reference point **RP** is an entry location point **EP** that is at or proximate the location where the planned trajectory line **PTL** intersects an entry surface of the body **B.** The visualization plane **VP** can be selected and scanned. In accordance with some embodiments, the guide axis **GA** is determined by tracking the fiducial array **1164a** or **1194a (****Figs. 17****,** **27C****).** In some embodiments, at least two different points on the tracker probe **1160** or **1190** or the targeting cannula **150** can be tracked or identified.

According to some particular aspects, two axially spaced apart acquisition planes **(AP1** and **AP2)** may be selected, the planes **AP1** and **AP2** being relatively oriented and positioned so that they intersect the guide device **1160, 1190** at different points (a first guide component point **GCP1** and a second guide component point **GCP2,** respectively) along the guide axis **GA.** Scans may optionally be taken along each acquisition plane **AP1, AP2** to track the position of the guide device in each plane **AP1, AP2.** From these two points, the orientation and position of the guide axis **GA** is determined.

According to some aspects, the guide device **1102, 1160, 1190** or **150** can be tracked using a camera based and/or EM based tracking device or devices such as LEDs, microcoils, or other suitable devices without requiring ongoing image scans of an imaging system such as an MRI or CT Scanner, to provide a planned trajectory line PTL.

Using the information from the tracking devices and/or scan(s) of the acquisition planes **AP1, AP2,** the orientation of the guide axis **GA** is programmatically determined. A controller **30p (****Fig. 1****)** of the IGS system **10** can extrapolate the guide axis **GA** and determine the location **GPP** of the guide axis' **GA** intersection with the visualization plane **VP.** According to some embodiments, the point of intersection **GPP** between the guide axis **GA** and the visualization plane **VP** is not a point on the guide device **1102, 1160, 1190** or **150.** The controller **30p** may determine or derive the location of the guide axis projected point **GPP** by mathematical calculation and/or any other suitable method.

The controller **30p** can provide a displayed image including representations of the sighting point (*e.g.,* the target point **TP**) and the guide axis projected point **GPP.** The display and tracking may be used by the operator to align the guide axis **GA** with the planned trajectory line **PTL (****Fig. 7****).** **Figs. 8A-C** show the guide device **1102, 1160, 1190** or **150** in a trajectory frame **100** as aligned with the planned trajectory line **PTL.**

In the methods as just described, the planned trajectory line **PTL** and the guide axis **GA** may not necessarily share a point (such as the pivot point **PP**) unless and until the planned trajectory line **PTL** and the guide axis **GA** are aligned. For example, according to some embodiments, the guide axis **GA** is translated (in the X-Y plane) relative to the planned trajectory line **PTL** by adjusting the setting(s) of one or both of the moving plates **118₁** and **118₂** of the X-Y table **132 (****Fig. 9A****).** In some embodiments, the orientation of the guide axis **GA** can also be adjusted by pivoting the guide axis **GA** about a pivot point (*e.g*., located at the reference point **RP**). As indicated by the translation direction arrow **D,** the guide device **5** can be linearly translated in an X and/or Y direction relative to the patient body **B** until the guide axis **GA** intersects the reference point **RP.** If necessary, the orientation of the guide device **5** can be adjusted to bring the guide axis **GA** into alignment with the planned trajectory line **PTL.** In some embodiments, the direction **D** is substantially perpendicular to the guide axis **GA.**

In some aspects, the system **10** can include one or more software modules that can automate or carry out aspects of the invention, including the navigation, tracking and calculating the planned trajectory line **PTL.** The modules can include data processing systems, digital signal processors and computer program products in accordance with aspects of the disclosure.

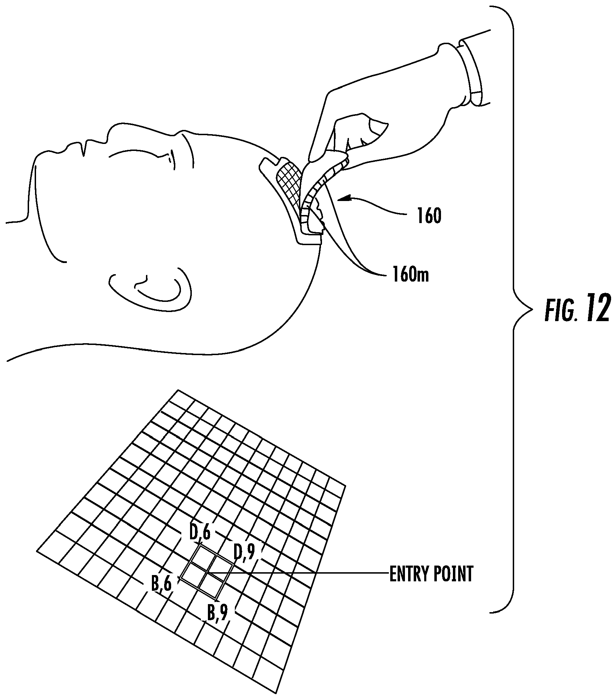

**Figs. 9A-9C****,** **10****,** **11A** and **11B** illustrate a trajectory frame **100.** **Fig. 12** illustrates a grid **160.** These devices are described in U.S. Patent No. 8,340,743. The trajectory frame **100** can include a support column (e.g., guide) **1102** that releasably holds one or more tools **T.** The trajectory frame **100** can have alternate or include additional materials or members that are image visible when used for non-MRI imaging modalities such as camera-guided systems, EM-guided systems and/or CT imaging systems.

In some aspects, the CT or MRI imaging modality fiducials **50F (****Figs. 9A-9C****)** and grid material **160m (****Fig. 12****)** for example, can include CT visible materials or hybrid materials that are visible in both MRI and CT images, for example.

In some aspects, the imaging fiducials **50F** (where used) can include or be doped with a substance having a high atomic number (Z), such as barium, titanium, iodine, silver, gold, platinum, iodine, stainless steel, titanium dioxide, etc. that provide good contrast on a CT or other radiographic imaging system. The fiducials **50F** can include gadopentatate dimeglumine, gadoteridol, ferric chloride, copper sulfate, or any other suitable MRI contrast agent, such as described in chapter 14 of Magnetic Resonance Imaging, 2nd ed., edited by Stark and Bradley, 1992.

In some aspects, the fiducials **50F** can be a solid material doped with a substance that provides good contrast using a first imaging modality (e.g., CT). A hygroscopic outer coating can be formed thereupon. The coating permits soaking up a fluid that provides a good contrast using a second imaging modality (e.g., MRI).

In some aspects, the trajectory frame **100** and/or other cooperating tools **T** such as, for example, a tracking probe **1160** or **1190** or **1500** or targeting cannula **150** can include fiducial markers **350F.** These fiducial markers **350F** can be tracking (e.g., reflective) optical or EM markers and/or fiducials.

In some aspects, the tracking fiducial markers **350F** comprise reflective members such as, for example, spherical locators **350s** and may have a body, coating, tape and/or outer surface that is reflective of light or other electromagnetic energy. Consequently, it is also locatable by the operating room camera **10C (****Figs. 1****,** **29****)** in an optical positioning system that is coupled to the image-guided workstation **30** (e.g., during patient registration). In one such example, the outer surface of the tracking members **350s** includes light-reflective microspheres (e.g., embedded in an adhesive covering). In another such example, the outer surface of the tracking members **350s** can include or be covered with a reflective coating or material, such as, for example, an adhesive-backed light-reflective tape, such as SCOTCHLITE® 9810 Reflective Material Multipurpose Tape sold by Minnesota Mining and Manufacturing Co. ("3M®"), of Saint Paul, Minn. Further examples of reflective tracking members **350s** are discussed below with respect to **Figs. 17-29****.**

**Figs. 10** and **11A** illustrate that the optical fiducial markers **350F** can be arranged to reside in an open space bordered by an inner perimeter of toroidal shaped image fiducials **50F** arranged so that a line drawn through a center of each image fiducial **50F** and or **350F** forms a circle **C.** The centers (centroids) of the tracking members (e.g., reflective spheres) **350s** can be concentric with respective fiducials **50F.** This may provide for a more direct concordance comparison of calculation of grid entry and/or trajectory and/or identification of registration errors or offsets.

**Fig. 11B** illustrates that the trajectory frame **100** can have a base **110** that includes co-planar ears **1117** spaced apart about the patient access aperture **112** interior to the base **110** that can hold the fiducials **350F, 50F** on an upper surface thereof. The ears **1117** can include a projecting post **1117p** that can allow a spherical member to be attached to so that the spherical member extends above an upper/outer surface of the adjacent fiducial **50F.** The spherical fiducials **350s** can have a reflective coating and may be passive spheres such as those available from Northern Digital Inc. (ndigital.com) as NDI passive spheres that attach via snap-on attachment to the posts **1117p.**

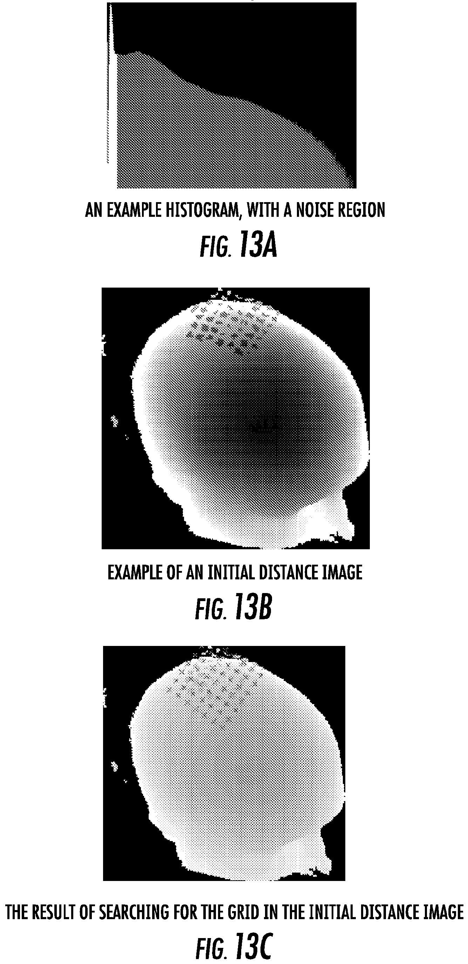

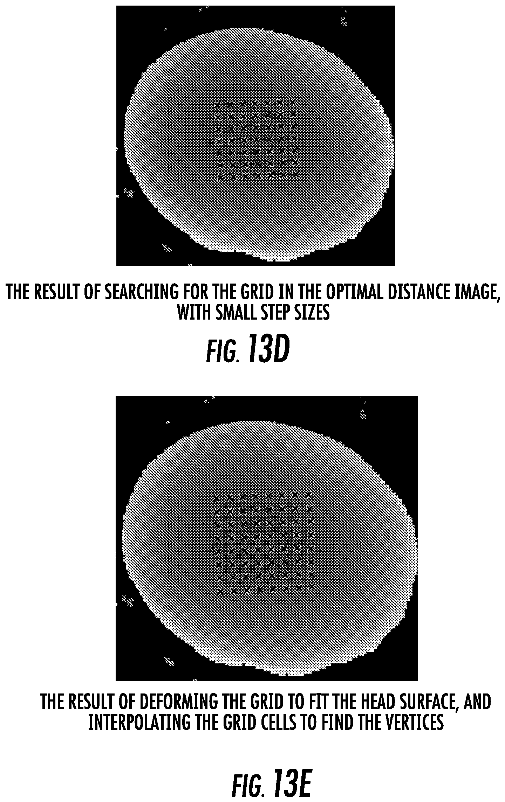

**Figs. 13A-13E** illustrate image data interrogation to identify location and orientation of the grid **160 (****Figure 12****).**

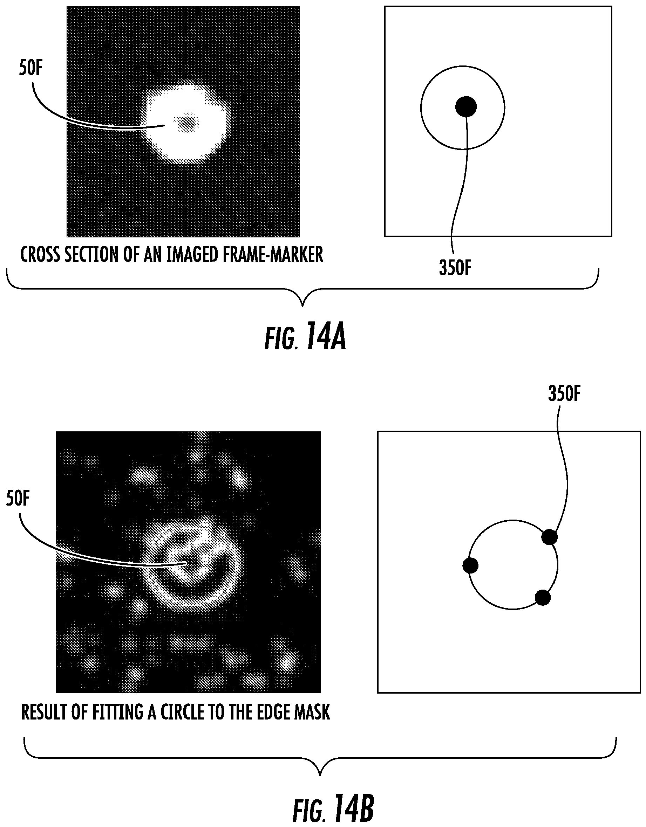

**Figs. 14A** and **14B** schematically illustrate exemplary side by side comparisons of location of the trajectory frame **100** may be calculated and/or shown using the different respective fiducials **50F** and **350F,** respectively.

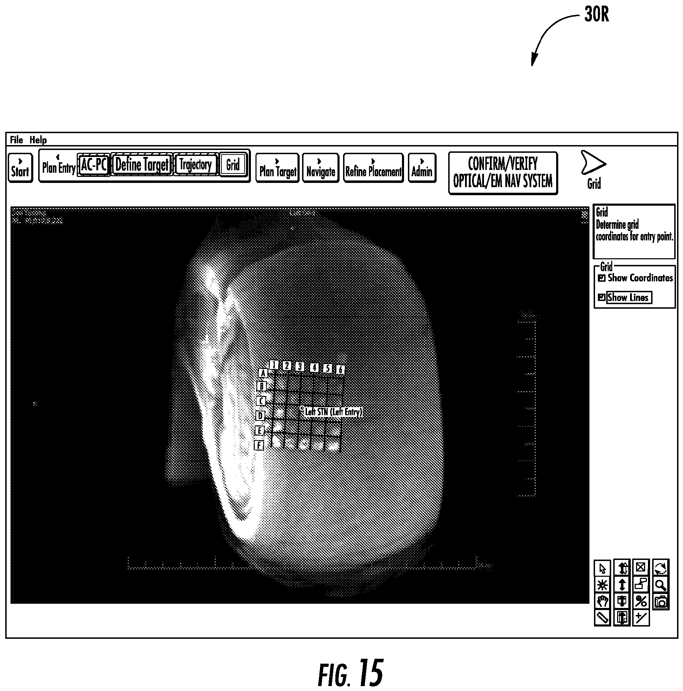

**Fig. 15** illustrates the "Verify" or "Confirm" UI **30R** for the optical and/or EM navigation systems **10** that can generate a separate image for a trajectory and/or calculate grid entry coordinates using a deformable grid based on grid **160** and fiducial image visible material **160m (****Fig. 12****).**

It is noted for completeness that although the use of a grid to plan a target entry with entry coordinates is contemplated for some systems not all systems need use this feature or operation, which may be determined in other ways.

The system **10** can include an imaging device or scanner **10S** that can be used to acquire pre-, intra-, or post-operative or (near) real-time image data of a subject, such as a patient. It will be understood, however, that any appropriate subject can be imaged and any appropriate procedure may be performed relative to the subject and camera IGS systems can be used. The scanner may be a closed bore CT scanner or an O-arm® imaging device sold by Medtronic Navigation, Inc. having a place of business in Louisville, Colorado, USA. The scanner **10S** may have a generally annular gantry housing and an image capturing portion. The image capturing portion may include an x-ray source or emission portion and an x-ray receiving or image receiving portion located generally or as practically possible 180 degrees from each other and mounted on a rotor (not illustrated) relative to a track or rail. The image capturing portion can be operable to rotate 360 degrees during image acquisition. The image capturing portion may rotate around a central point or axis, allowing image data of the patient to be acquired from multiple directions or in multiple planes. The scanner **10S** can include those disclosed in U.S. Pat. Nos. 7,188,998; 7,108,421; 7,106,825; 7,001,045; and 6,940,941.

For a discussion of an example of a system **10** that includes EM tracking and registration methods, see, U.S. Patent No. 8,238,631. The EM tracking system may include the STEALTHSTATION® AXI EM™ Navigation System, sold by Medtronic Navigation, Inc. having a place of business in Louisville, Colorado; or can be an EM tracking system described, for example in one or more of U.S. Patent Application Publication US2005/0085720, filed Sept. 15, 2004, and entitled "METHOD AND APPARATUS FOR SURGICAL NAVIGATION"; U.S. Patent No. 5,913,820, entitled "Position Location System," issued June 22, 1999; and U.S. Patent No. 5,592,939, entitled "Method and System for Navigating a Catheter Probe," issued January 14, 1997. It will be understood that the tracking system **82** may also be or include any appropriate tracking system, including a STEALTHSTATION® TREON® or S7™ tracking systems having an optical localizer, similar to the optical localizer **94,** and sold by Medtronic Navigation, Inc. of Louisville, Colorado. Other tracking systems include an acoustic, radiation, radar, etc. The tracking systems can be used according to generally known or described techniques in the above references.

As noted above, the systems **10** are configured so that hardware, e.g., one or more specific surgical tools, constitute a point of interface with the system (software or computer programs) because the circuit **30c** is configured with predefined tool data that recognizes physical characteristics of specific tool hardware.

As shown in **Figs. 9A-9C****,** a guide **1102** configured to hold one or more surgical tools can be attached to the trajectory frame **100.** The trajectory frame **100** can provide X-Y adjustment and pitch and roll adjustment.

The frame **100** can include a yoke **120** with control arcs **(****Figs. 9A-9C****)** that cooperate with a platform **130 (****Fig. 9A****)** to provide pitch and roll adjustments. The platform **130** has an X-Y table **132** that can allow for X-Y adjustments of the trajectory. For additional discussion of suitable trajectory frames **100,** see U.S. Patent Application Publication US2008/0306375 and US2009/0112084

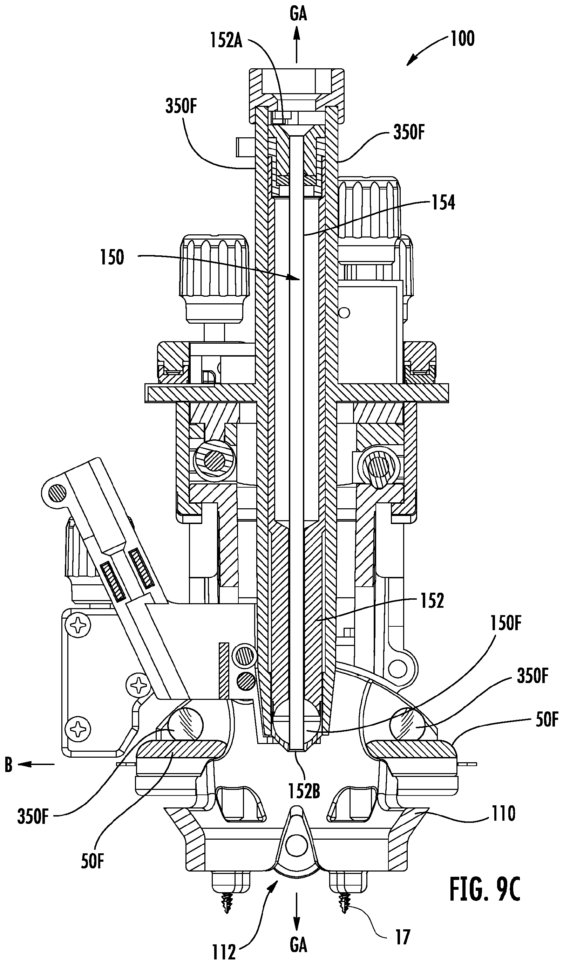

Referring to **Fig. 9C****,** where used, a targeting cannula **150** held in the trajectory frame **100** can include a through passage. The distal end **152B** of the targeting cannula can have an image fiducial marker **150F,** shown as a substantially spherical or round (cross-section) marker shape. The proximal end **152A** can be configured with a fluid filled channel concentric **152** with the passage **154** and the channel can be in fluid communication with the cylindrical fiducial marker **150F.** Along the axis of the cannula, there is the lumen or passage **154** through which another device can be slidably introduced and/or withdrawn, e.g., a stylet, imaging probe, therapy delivery or diagnostic device such as DBS stimulation leads for implantation, can be advanced into the brain or other target region.

Referring now to **Fig. 17****,** a trajectory frame **100,** according to embodiments of the present invention, is illustrated. The trajectory frame **100** can include a support column or guide **1102** that is configured to removably receive one or more devices of various sizes and configurations. The illustrated trajectory frame **100** is configured to be mounted to a patient's skull around a burr hole ring and over a burr hole, to provide a stable platform for advancing surgical devices, leads, etc., in the brain, as described above. However, a trajectory frame **100** according to embodiments of the present invention can be configured to be mounted to various portions of the body of a patient.

Again, as described for **Figs. 9A-9C****,** the illustrated trajectory frame **100** includes a base **110,** a yoke, **120,** a platform **130,** and a plurality of actuators **140a-140d.** The base **110** has a patient access aperture **112** formed therein, as illustrated. The base **110** is configured to be secured (directly or indirectly) to the skull or scalp of a patient such that the patient access aperture **112** overlies the burr hole **10** in the patient skull. The base **110** can include a plurality of narrow, tapered members that can be driven into the skull of a patient to prevent the base **110** from moving but also or alternatively typically includes fasteners **17 (****Fig. 9C****),** such as screws, can then be used to secure the base to the skull of the patient, as described above. Where the trajectory frame is used for other body locations other body positional and/or attachment configurations may be used.

The yoke **120** is movably mounted to the base **110** and is rotatable about a roll axis RA. The platform **130** is movably mounted to the yoke **120** and is rotatable about a pitch axis PA. The illustrated platform **130** includes an X-Y support table **132** that is movably mounted to the platform **130.** The X-Y support table **132** is configured to move in an X-direction and Y-direction relative to the platform **130** and to a Z-direction defined by the longitudinal axis of the guide **1102.** An X-direction actuator **140c** is operably connected to the platform **130** and is configured to move the X-Y support table **132** in the X-direction. A Y-direction actuator **140d** is operably connected to the platform **130** and is configured to move the X-Y support table **132** in the Y-direction. A pitch actuator **140b** is operably connected to the platform **130** and is configured to rotate the platform **130** about the pitch axis PA.

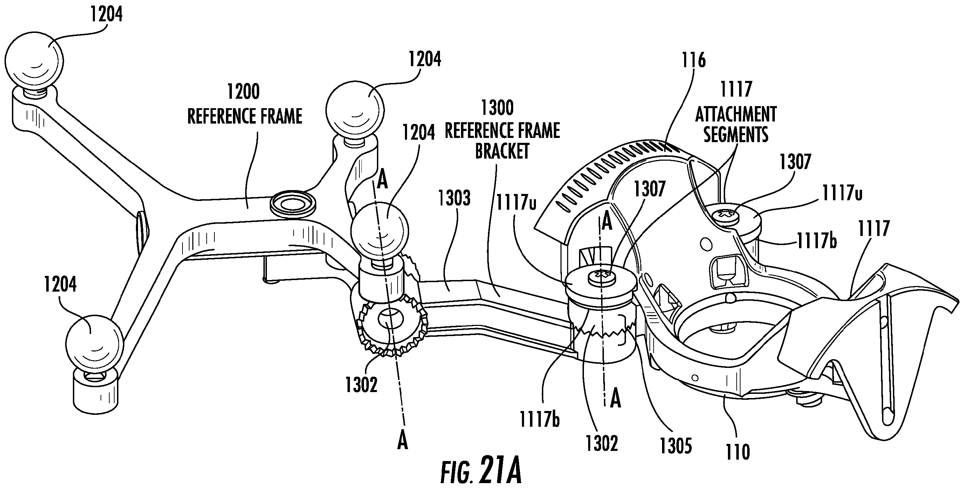

The actuators **140a-140d** are configured to translate and/or rotate the frame. When inserted within the guide **1102,** the targeting cannula **150,** tracking probe **1160** or **1190 (****Fig. 21A****,** **31A****)** and other devices inserted within the guide **1102,** are configured to translate in response to translational movement of the X-Y support table **132** and to rotate in response to rotational movement of the yoke **120** and platform **130** to define different axial intrabody trajectories extending through the patient access aperture **112** in the frame base **110.**

The trajectory frame guide **1102** is configured to removably receive various probes and/or tools, as described below. For example, the guide **1102** may have a larger diameter than conventional targeting cannula guides which, thereby allows for various devices to be utilized with the frame **100** that otherwise would not be able to do so.

In addition, the trajectory frame **100** can releasably serially and interchangeably hold more than one guide **1102** having different size internal diameters for receiving various devices of different sizes. In some embodiments, a single guide **1102** can be integral to the frame **100** and configured to receive different tools having different diameters. If the former, for example, a guide **1102** may have an internal diameter sized to receive a particular device therein. Another guide **1102** may have a larger or smaller internal diameter also sized to receive a particular device therein. To facilitate replacing one size guide **1102** with another, each guide **1102** may be removably and interchangeably secured to the X-Y support table **132.** For example, each guide may be threadingly secured to the X-Y support table **132.** However, other means for removably securing a guide **1102** to the X-Y support table **132** can be utilized.

The trajectory frame **100** allows for the adjustability (typically at least two degrees of freedom, including rotational and translational) and calibration/fixation of the trajectory of at least one of, and typically all of, a targeting cannula **150,** and a tracking probe **1160** and/or **1190 (****Figs. 17****,** **27A****)** and/or other probe or tool inserted through the or a respective guide **1102.**

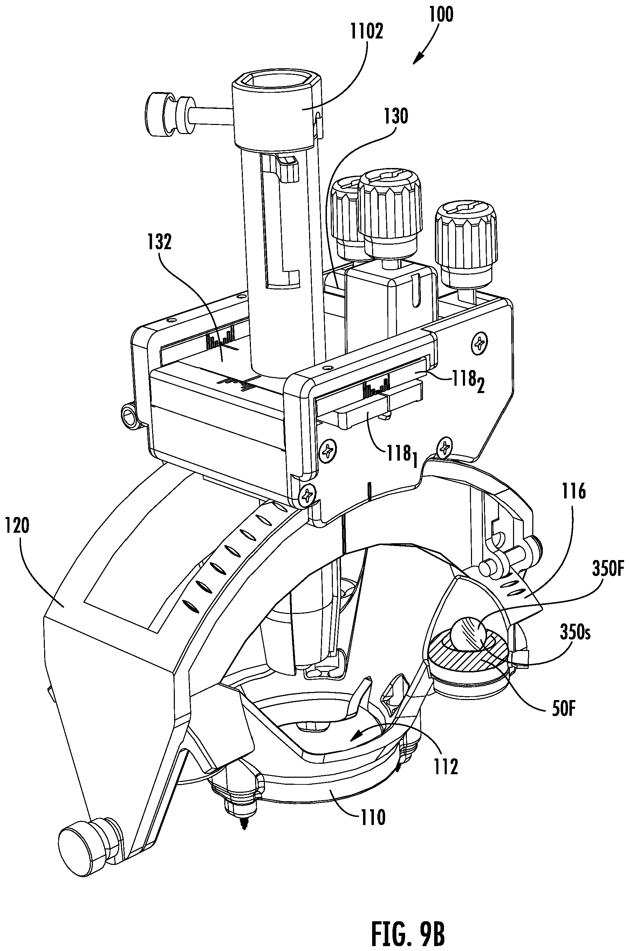

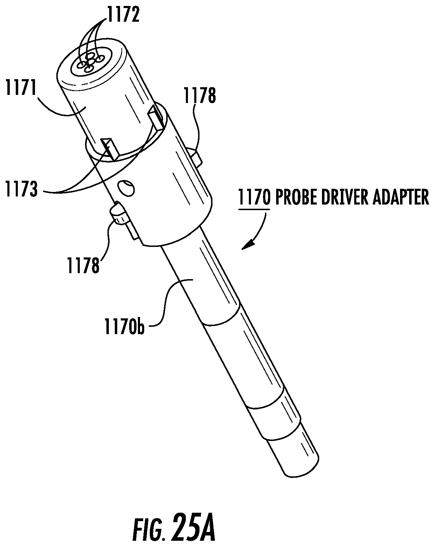

The trajectory frame **100** may optionally include fiducial markers **50F (****Fig. 9B****)** (when used for MRI or CT-image guided systems) that can facilitate registration of position in an image. The support column or guide **1102** can include one or more longitudinally and one or more laterally extending slots that slidably receive lugs of a tool **T,** e.g., lugs **1168** of a tracking probe **1160 (****Fig. 17****),** lugs **1178** of a probe drive adapter **1170 (****Figs. 25A-C****)** and/or lugs **1198** of a universal tracker probe **(****Figs. 27A-C****),** for attachment to the guide **1102** or an interchangeable respective guide **1102** held by the X-Y support table **132.** The tools are not required to be MRI-compatible as they may be used for non-MRI surgical image/camera tracking and/or guided systems but may be MRI-compatible and suitable for use in different imaging systems.

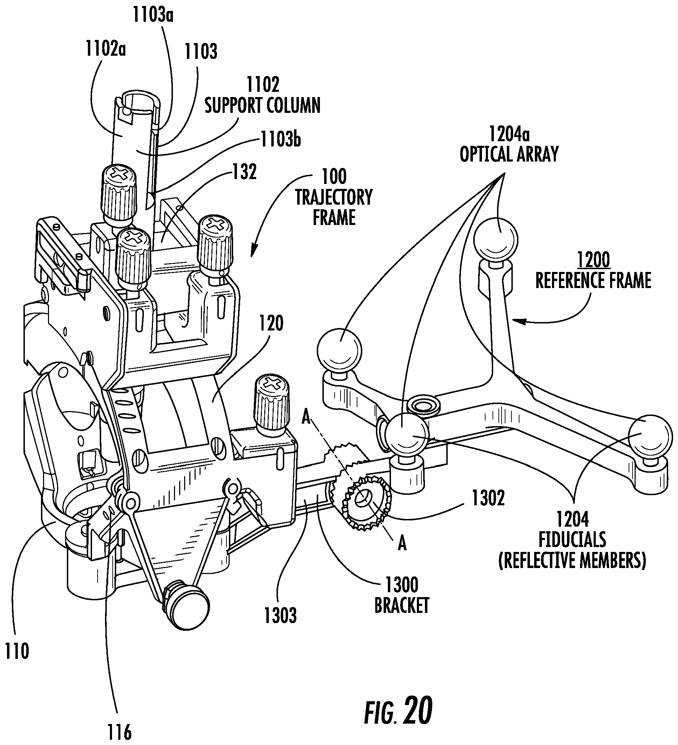

The guide **1102** has opposite proximal and distal end portions **1102a, 1102b.** In some embodiments, the proximal end portion **1102a** contains threads while in other embodiments threads are not required (as shown in **Fig. 20****,** for example). These threads, where used, can be molded or machined into the guide **1102,** as would be understood by those skilled in the art of the present invention. The threads can be configured to threadingly engage a correspondingly threaded cap to secure a tool within the guide **1102,** and to allow for quick removal.

As shown in **Fig. 20****,** the guide **1102** includes at least one downwardly extending slot **1103,** shown as a pair of opposing slots, formed in the proximal end portion **1102a,** thereof. Each slot **1103** can include an upper transverse slot or ledge portion **1103a** and a lower transverse slot or ledge portion **1103b** that are configured to engage lugs. The respective lugs of different tools can cooperate with the slots **1103** to allow the tool to be inserted within the guide **1102.** By rotating the tool, the lugs cooperate with the upper ledge portions **1103a** and the tool can be positioned at a first or upper position. By inserting the tool further within the guide **1102** and then rotating it, the lugs cooperate with the lower ledge portions **1103b** and the tool can be securely held at a second or lower position.

The elongated slots **1103** merge into spaced-apart transversely extending upper ledge portions (e.g., slots) **1103a** and transversely extending lower ledge portions (e.g., slots) **1103b.** The distance between the upper ledge portions **1103a** and the lower edge portions **1103b** is typically between about 0.25 inches (6.4mm) and about 5.0 inches (127mm).

Referring now to **Figs. 17-29****,** the trajectory frame **100** is shown configured to be used for camera-guided guided systems **10C,** **Fig. 29** or EM guided systems **10EM (Fig. 35).** The trajectory frame **100** and cooperating components or tools may be configured for use with "asleep" or "awake" neurological (e.g., brain) surgical systems.

**Fig.** 17 illustrates the trajectory frame **100** (e.g., also known as a trajectory guide) with a guide **1102** (e.g., also known as a support column) holding an optical tracking probe **1162** with reflective members **1164** according to embodiments of the present invention. This tracking probe **1162** includes a plurality of spaced apart fiducials **350F,** typically reflective members which may be spherical or have other shapes, arranged as an array of reflective members **1164a.** The array **1164a** can be positionally adjusted but the individual reflective members **1164** are typically held in a fixed geometric relationship relative to each other. The array **1164a** can include four (or more) reflective members, shown as spheres, which can allow for AC-PC image views. The reflective members **1164** can have a reflective surface and may have a reflective tape or coating. In some particular embodiments, the reflective members **1164** may be passive spheres such as those available from Northern Digital Inc. (ndigital.com) as NDI passive spheres that attach via snap-on posts.

The tracking probe **1162** can be held in an elongate tracking probe mount **1160** that can include lugs **1168** that releasably attach to the guide **1102** that is attached to the X-Y support table **132.** The tracking probe mount **1160** includes upper and lower ends, **1160a, 1160b,** respectively. The lower end **1160b** is typically held in the guide **1102** so that it is positioned to extend below the bottom or distal end of the guide **1102b** to be able to bottom out or contact the skull or scalp of the patient to define a desired trajectory. The optical tracking probe **1162** can be held above the top end **1160a** of the mount **1160.**

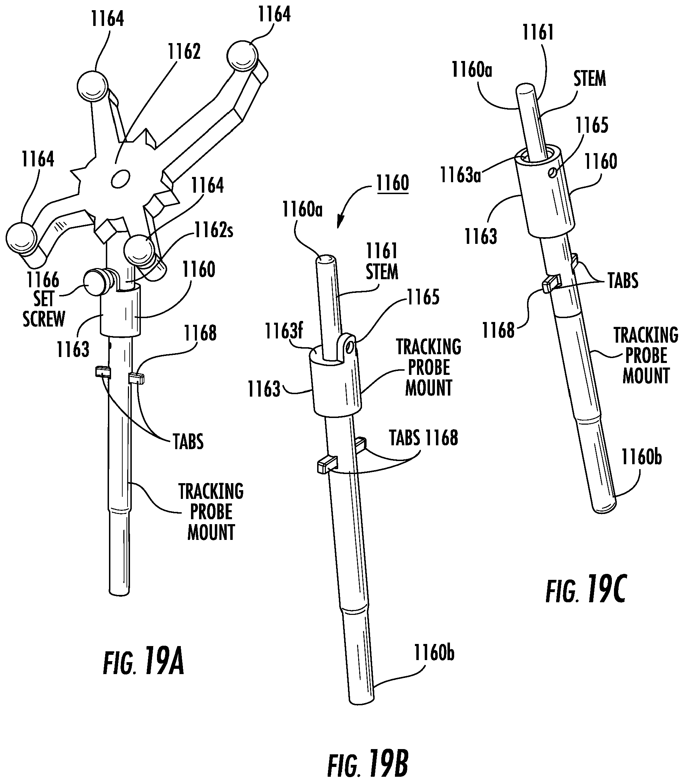

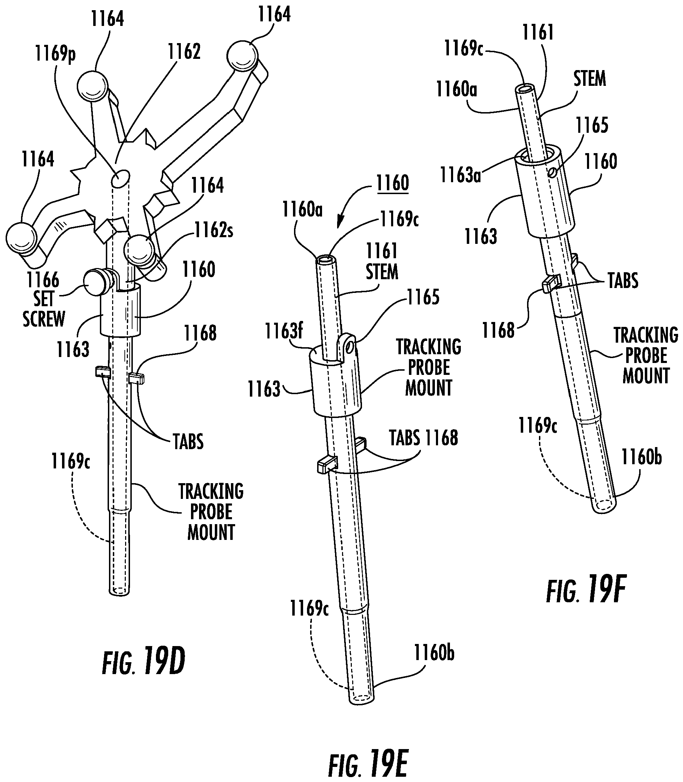

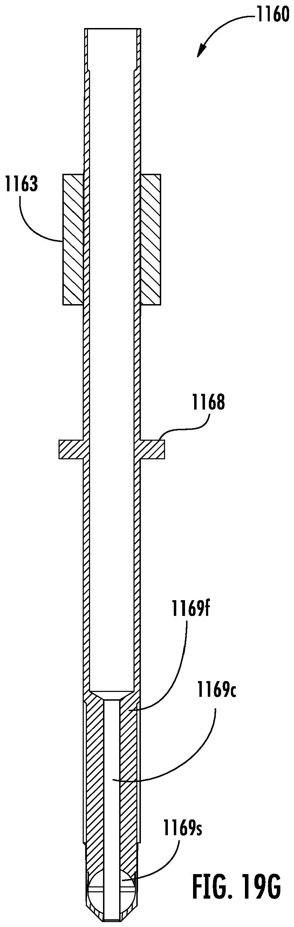

**Fig. 19A** shows the optical array **1162** with an exemplary mount **1160.** **Figs. 19B** and **19C** illustrate two alternate configurations of the mount **1160,** each having laterally extending lugs **1168** and a collar **1163** that surrounds an upwardly extending stem **1161** that slidably extends into a bore in a downwardly extending support member **1162s** of the optical array **1162.** **Fig. 19B** illustrates a flat closed upper surface **1163f** of the collar **1163** that can abut a flat lower surface of the optical array support member **1162s.** **Fig. 19C** illustrates that the collar **1163** can have an open annular channel that receives a lower end of the optical array support member **1162s.** The lower portion of the collar **1163** can engage the top of the guide **1102a.**

**Figs. 19A-19C** illustrate that the collar **1163** can include at least one aperture **1165** that allows for a fixation member **1166** to extend therethrough to lockingly engage the lower end portion of the optical array support member **1162s.** Although shown as one fixation member and aperture, a plurality of circumferentially spaced apart members/apertures may be used. Also, other fixation configurations may be used including, for example, clamps, frictional engagement grips, bayonet fittings or other configurations that lock the device **1162** in position on or in the mount **1160** so that the optical array **1162** does not flex or move other than with the mount **1160** in the guide **1102.**

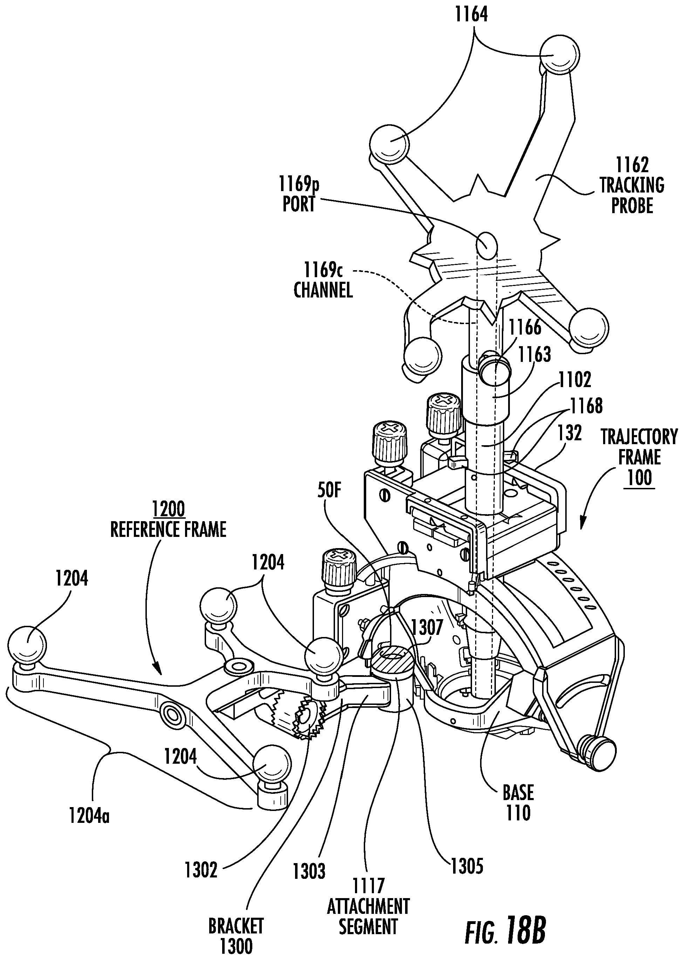

**Figs. 19D-19G** illustrate another embodiment of the tracking probe **1162** and tracking probe mount **1160.** In this embodiment, the tracking probe mount **1160** can optionally include a through channel **1169c** and the tracking probe **1162** can include an aligned port **1169p.** Different devices can optionally be guided down through the channel **1169c** for a desired trajectory provided by the tracking probe mount **1160.** The tracking probe mount **1160** can include image fiducials **50F (****Figure 9A****)** that may include MRI and/or CT visible segments, such as fluid-filled segments, which may optionally comprise fluid filled wall segments (which may be concentric to the channel) **1169f** surrounding all or part of the open channel **1169c.** The MRI/CT visible segments (image fiducials) can comprise a fluid-filled spherical member **1169s** at a distal end portion of the tracking probe mount **1160.** The trajectory frame **100** may include fluid-filled MRI/CT visible fiducials **50F (****Fig. 18B****).**

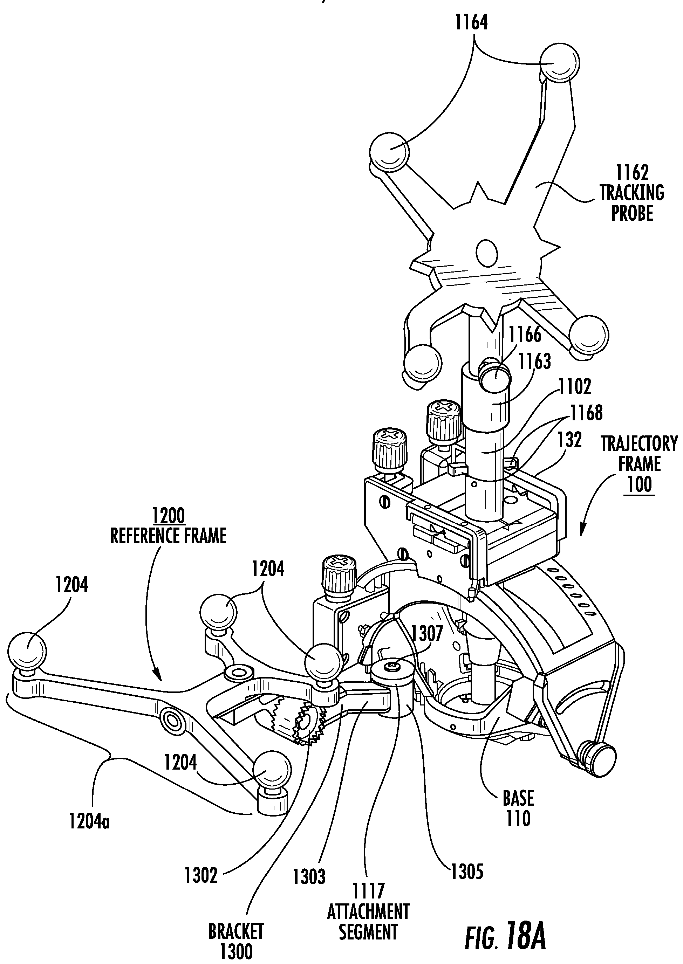

**Figs. 18A** and **18B** illustrate that the trajectory frame **100** can also be configured to hold both the optical tracking probe **1162** and an optical reference frame **1200.** The optical reference frame **1200** can include an array of reflective members **1204a,** typically four spherical reflective members but other shapes can be used such as those discussed above with respect to the tracking probe **1162.**

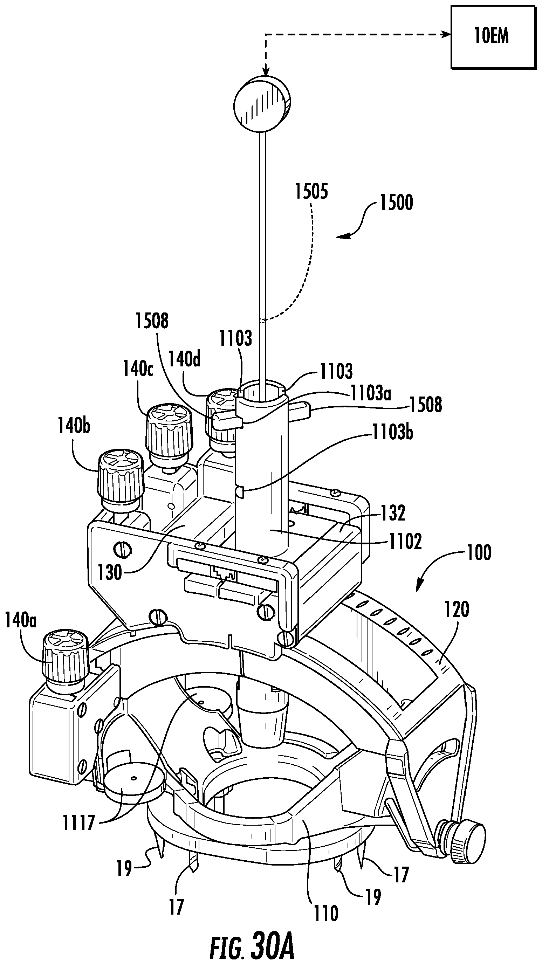

In some embodiments, a first planned trajectory can be generated using a camera-based or EM based navigation/tracking system with a corresponding tracking probe **1162** or **1500 (****Fig. 29** or **31****),** for example. A confirmation or concordance trajectory can be calculated (or fine adjustments made) using the CT and/or MRI image fiducials **50F** as a review/oversight or confirmation or adjustment of the trajectory calculated using the optical tracking probe mount **1160** or the EM tracking probe mount **1510 (****Fig. 30A****).** The CT/MRI image fiducials **50F** can reside on the tracking probe **1162,** tracking probe mount **1162** or EM probe **1500,** and/or trajectory frame **1100.** In other embodiments, a targeting cannula **150 (****Fig. 9C****)** can be interchangeably placed in the bore of the guide **1102** after removing the tracking probe **1162,** alone, or the tracking probe **1162** with the tracking probe mount **1160,** and used for the concordance trajectory evaluation. In any event, using the CT or MRI Scanner to supplement or confirm the trajectory can reduce any required imaging time from an MRI and/or CT Scanner and yet provide a precise trajectory.

The reference frame **1200** can be held by a bracket **1300** that is attached to the trajectory frame **100.** The reference frame **1200** can extend a distance beyond an outer surface of the platform **132** with the fiducials **1204** in a fixed geometric pattern that may extend along a common plane or at different planes and can allow for AC-PC image views. The reference frame **1200,** when attached to the trajectory frame **100,** may be particularly suitable for "awake" brain surgical procedures to track patient movement. For "asleep" neuro surgeries, the reference frame **1200** may be attached to the trajectory frame **100** and/or a head fixation frame that holds a patient's head in a stationary position (not shown).

The reference frame **1200** can be configured to extend from a defined one of a left side or right side or can be configured to be able to extend from a selected either side of the trajectory frame **100,** when looking from a front of a patient. The bracket **1300** can have a dedicated left side attachment configuration, a dedicated right side configuration or a configuration that can be used to extend off either side of the trajectory frame **100.** Two trajectory frames may be used for bilateral procedures, each with a respective reference frame **1200** (not shown).

The bracket **1300** can include at least one starburst connector **1302.** The starburst connector **1302** can allow for positional adjustment of the reference frame **1200** relative to the patient and/or base **110** of trajectory frame **100.** **Figs. 18A****,** **18B****,** **20** and **21A** illustrate that the at least one starburst connector **1302** can include a starburst connector that resides closer to the reference frame **1200** than the base **110.** The rotational or swivel axis **A-A (****Fig. 20****)** can extend perpendicular to the length dimension of the arm or link **1303.**

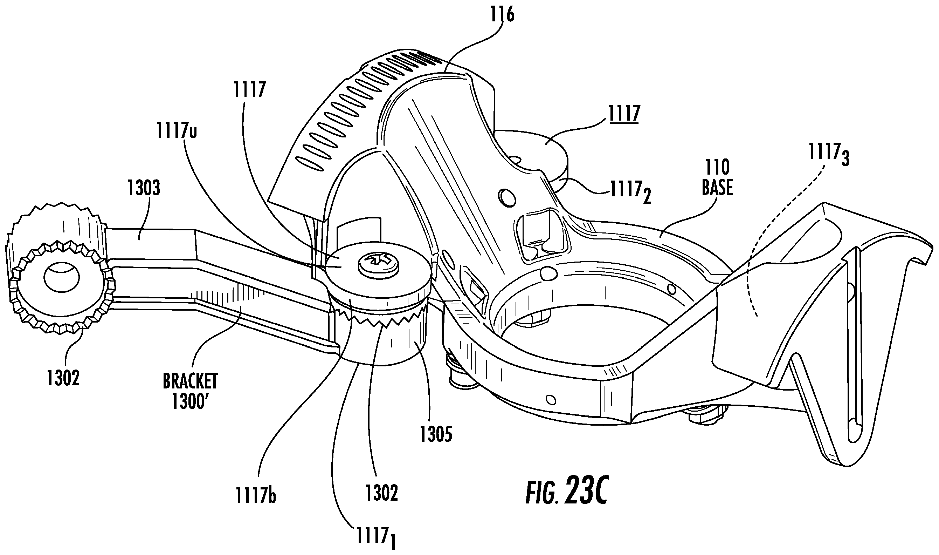

The trajectory frame **100** can have three concentric ears **1117₁, 1117₂, 1117₃ (****Figs. 10****,** **23C****),** positioned about the base **110** and can have two that reside closer together than a third, e.g., the ears **1117** can be asymmetrically oriented about a circle drawn through the centers of the ears **1117.**

As shown in **Figs. 18A****,** **18B****,** the trajectory frame **100** can have one or more attachment ears **1117** that extend outside the base **110,** above the bottom surface of the base **110** and under the platform **130** and/or support table **132.** The one or more ears **1117** can be a plurality of ears **1117** that extend outside a perimeter of the base. The ears **1117** can be the surfaces that support MRI fiducials **50F (****Fig. 9A****,** **18B****)** when used in MRI and/or CT image guided systems or when used with images from these types of imaging systems to confirm placement or trajectories (e.g., the concordance evaluation discussed herein). The ears **1117** can have upper and lower surfaces, **1117u, 1117b (****Fig. 21A****),** respectively, one or both of which can be planar.

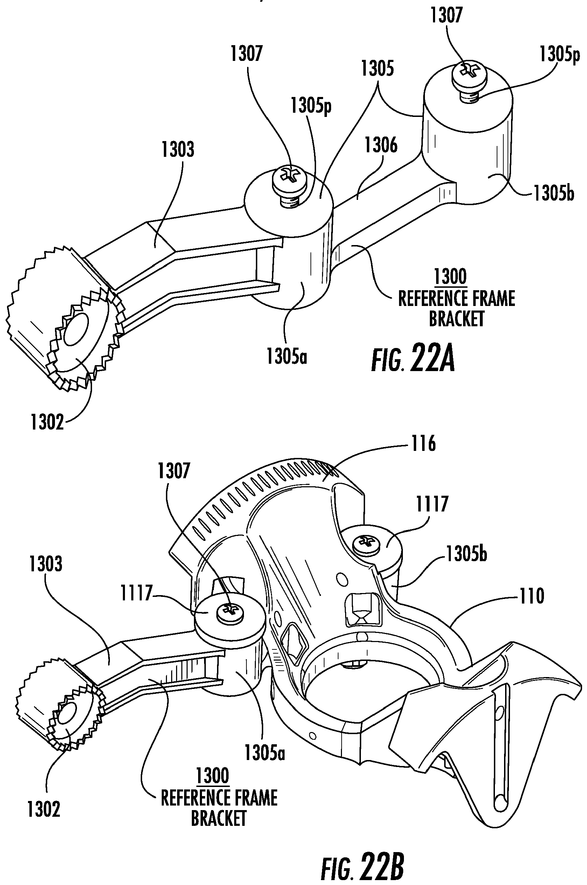

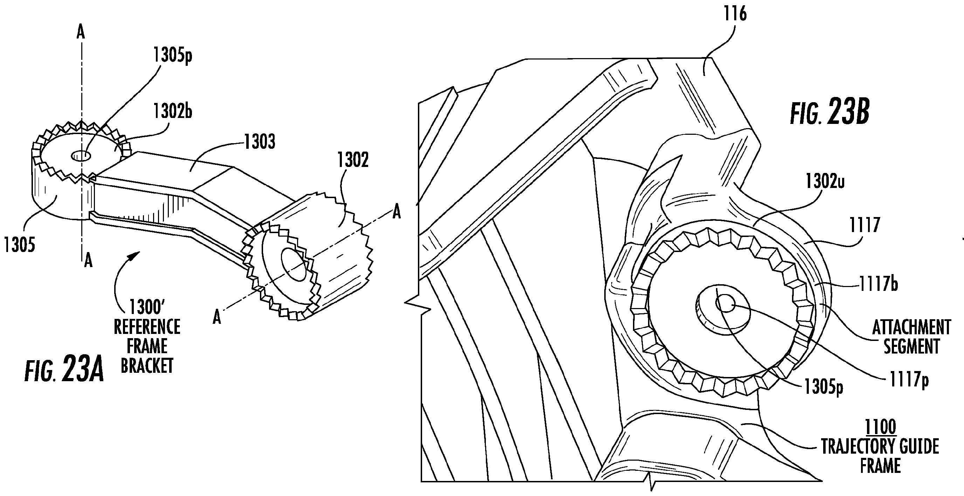

As shown in **Figs. 18A****,** **18B** and **21A****,** the bracket **1300** can have at least one upright segment **1305** that is attached to a respective ear **1117.** As shown in **Figs. 18A****,** **18B** and **21A****,** a pin or screw **1307** can be used to attach the upright segment **1305** to the ear **1117** and the pin or screw **1307** can extend down through the ear **1117,** typically through a pin or screw aperture **1117p** and connector aperture **1305p (****Figs. 22A****,** **23A****).** However, the upright segment **1305** can also be bonded, glued, adhesively attached and/or ultrasonically inserted into or moldably attached or otherwise integrated into a respective ear **1117.**

The upright segment **1305** can have a top surface that abuts the bottom surface of a respective ear **1117b.** Although not shown, the upright segment **1305** can have prongs or overlying wall segments that reside above and below the ear **1117** with a channel that receives and holds the ear **1117** therebetween for attachment or the upright segment **1305** can reside on the upper surface of the ear and be attached to the ear **1117.** The upright segment **1305** can support an outwardly extending linkage or arm **1303** that places the reference frame **1200** at a desired closely spaced apart position from the base **110.** The arm or linkage **1303** can have a length that is typically between about 0.25 inches (6.4mm) to about 3 inches (76mm) and can raise up as it extends outward away from the base **110.**

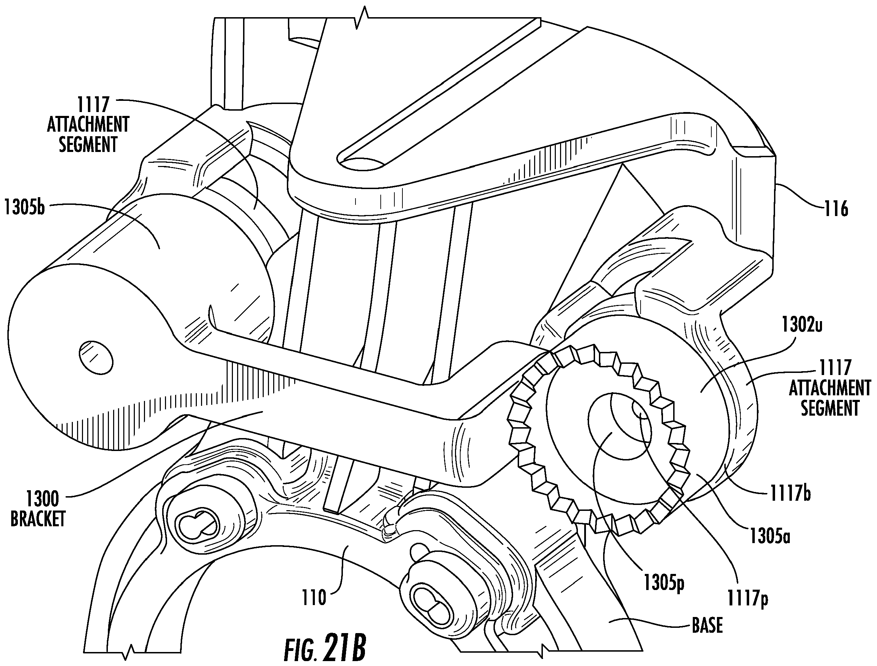

**Figs. 21A****,** **21B****,** **22A** and **22B** illustrate one example of a bracket **1300.** In this embodiment, the bracket **1300** can have two upright segments **1305,** e.g., first and second segments **1305a, 1305b,** each of which can attach to spaced apart ears **1117.** Typically, the upright segments **1305a, 1305b** are connected by a laterally extending straight arm **1306** that is at a level below the upper surface of the first and second segments **1305** and that can be below that of the outwardly extending arm or linkage **1303.** The bridging or connecting arm **1306** can extend proximate to but under the arcuate arm **116.** The dual upright supports may provide a more stable attachment for the arm **1303** and/or cantilevered reference frame **1200.**

The bracket **1300** can include a plurality of the starburst connectors **1302,** including one forming part of the upright segment **1305** proximate the base **110** and one residing further away from the base **110** and proximate the reference frame **1200.** **Fig. 21B** shows the upper component of the starburst connector **1302u** attached to the underside of the ear **1117b.** Each can have an axis **A-A** that is perpendicular to the other. The axis **A-A** of the upright segment **1305** can allow for front to back or right to left positional adjustment. The axis **A-A** of the connector **1302** proximate the reference frame **1200** can allow up and down adjustment of the arm **1303** and thus, the reference frame **1200.**

**Figs. 22A** and **22B** illustrate that the upright segments **1305** are not required to have starburst connectors **1302.** However, where used, typically only the upright segment closest to the arm or linkage **1303** will have the starburst connector **1302** for rotational adjustability of the arm or linkage **1303.** In any event, the bracket segments **1305a, 1305b** can be reversed to attach to the other ears **1117** so that the link **1303** extend from a desired side of the trajectory frame.

**Figs. 23A-23C** illustrate another exemplary bracket **1300'.** In this embodiment, a single upright segment **1305** is used to attach to a single ear **1117.** **Fig. 23A** illustrates the bracket **1300'** with the lower component of the starburst connector **1302b** while **Fig. 23B** illustrates the upper component **1302u** attached to an underside **1117b** of the ear **1117** of the trajectory frame **100.** Again the two axis of rotations **A-A** can be orthogonal to each other. The upright segment **1305** can be attached to the first, second or third ear **1117₁-1117₃.**

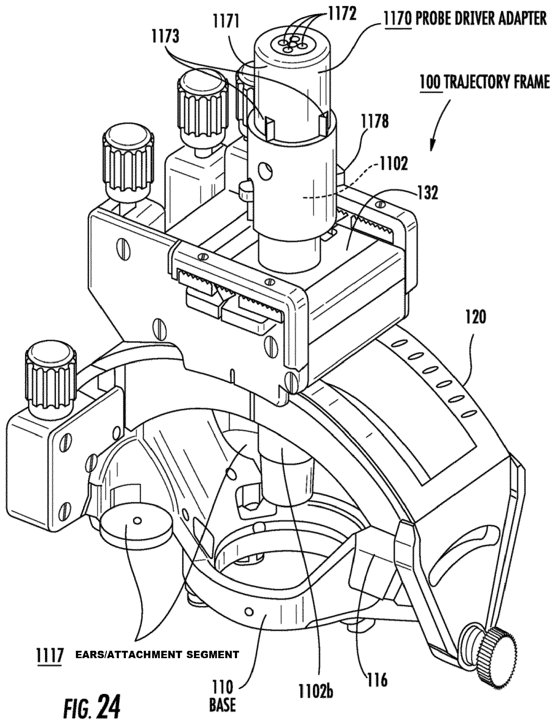

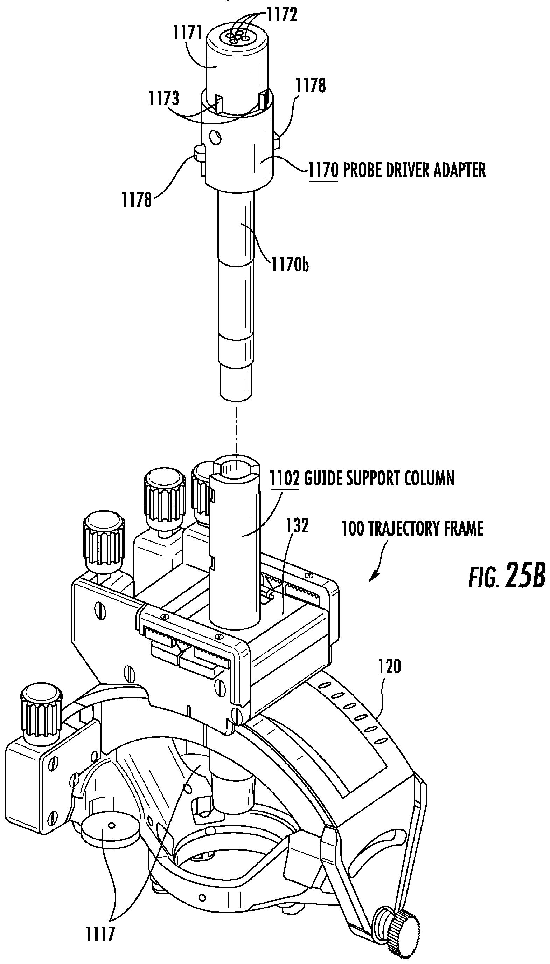

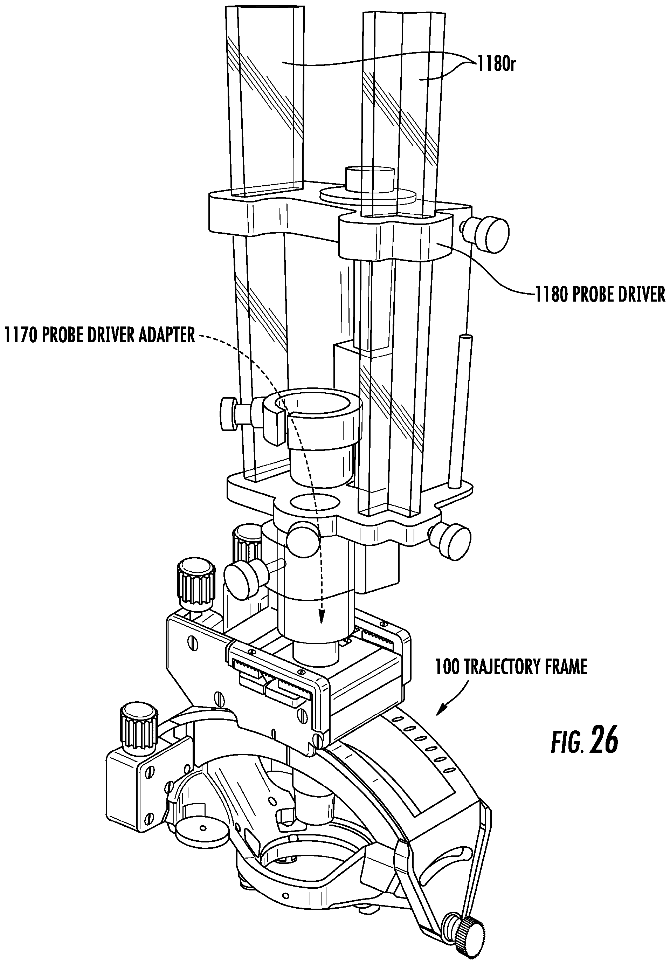

**Figs. 24****,** **25A** and **25B** show the trajectory frame **100** with the guide/support column **1102** releasably holding a microelectric (MER) probe driver adapter **1170,** typically used for "awake" brain surgeries, according to embodiments of the present invention. The probe driver adapter **1170** can hold the MER probe driver adapter body **1171** with microelectrode (entry) ports **1172** at a location that positions the microelectrode ports **1172** exposed above the adapter body **1171.** The adapter body **1171** can have outwardly extending lugs **1178** that engage the slots in the guide **1102.** The MER probe drive adapter body **1171** can have radially extending tabs **1173** that define a stop for the upper portion of the adapter body **1171** so that it extends a desired distance above the lugs **1178** and/or so that the driver engages the probe drive adapter at a desired position. The MER probe drive adapter body **1171** can matably engage a drive system **1180 (****Fig. 26****)** such as the NEXDRIVE® drive system with upwardly/outwardly extending rails **1180r (****Fig. 26****)** from Medtronic, Inc. As shown in **Fig. 26****,** the probe drive adapter **1170** resides between the rails **1180r** attached to a support frame of the probe driver system **1180.**

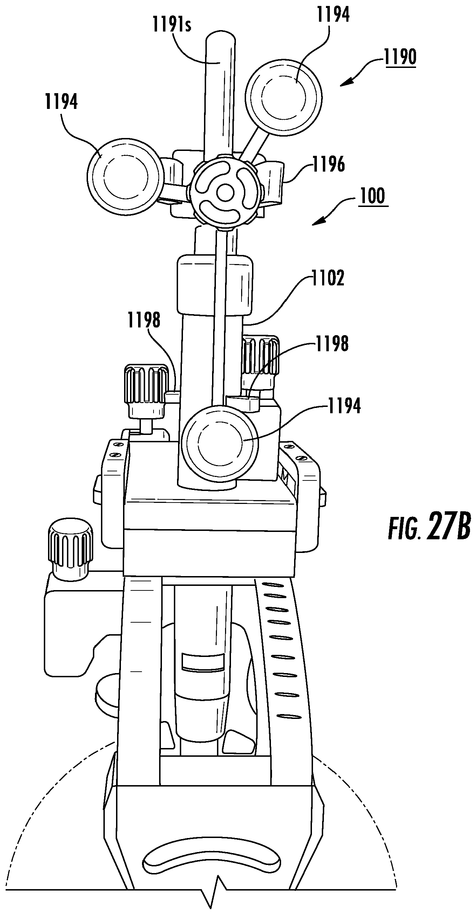

**Figs. 27A** and **27B** illustrate the trajectory frame **100** holding a universal tracker **1190** according to embodiments of the present invention. The universal tracker **1190** typically includes an array **1194a** of between 2-10 reflective members, typically two, three or four, although shown as three reflective members **1194.** As discussed with the other tracking probe **1162** above, the reflective members **1194** can have a reflective coating or tape and may optionally comprise passive spheres such as those available from Northern Digital Inc. (ndigital.com) as NDI passive spheres that attach via snap-on posts.

The universal tracker **1190** can be held in an elongate tracking probe mount **1190m** that can include lugs **1198** that releasably attach to the guide **1102** that is attached to the X-Y support table **132.** The tracking probe mount **1190m** includes upper and lower ends, **1190a, 1190b,** respectively. The lower end **1190b** is typically held in the guide **1102** so that it is positioned to extend below the bottom or distal end of the guide **1102b** to be able to bottom out or contact the skull or scalp of the patient to define a desired trajectory. The optical universal tracker reflective members **1194** can be held external of the mount **1190m.**

**Fig. 27C** shows the universal tracker **1190** with the reflective member array **1194a** for releasable attachment to a support column of the trajectory frame **100.** The lowest reflective member of the array **1194a** can be held at between about 5-6 mm above the lugs **1198,** in some embodiments.

**Fig. 27D** is a front view of the universal tracker **1190** without the optical array **1194a.** The tracker mount **1190m** includes an upwardly extending stem **1191s** that holds the optical array bracket **1196** and a collar **1192** that can engage the top of the guide **1102.**

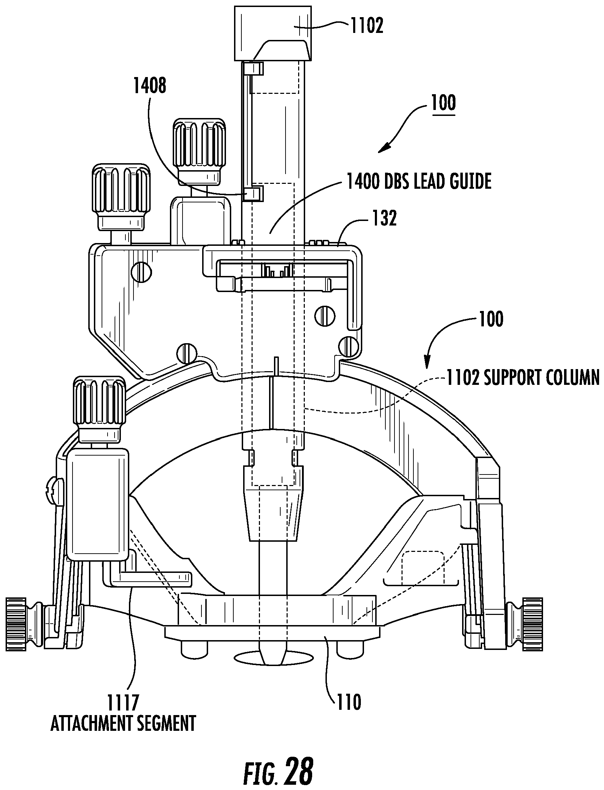

**Fig. 28** is a side view of a trajectory frame **100** illustrating the universal tracker **1190** shown in **Figs. 27A-D****,** replaced by a device (DBS lead) guide **1400** with lugs **1408** that engage guide (e.g., support column) **1102** according to embodiments of the present invention.

**Fig. 29** is a schematic illustration of a camera-based image guided system **10** with a software imaging/tracking module and camera tracking system **10C** that can be used with the trajectory frame **100** and various components discussed herein according to embodiments of the present invention.

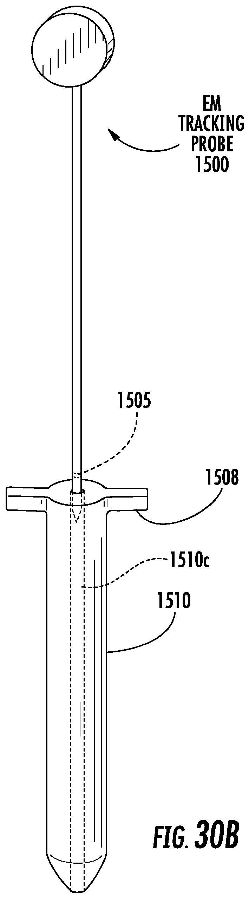

**Fig. 30A** illustrates a trajectory frame **100** cooperating with a EM tracking probe **1500** for EM-based tracking systems **10EM.** The tracking probe **1500** can releasably attach to the guide **1102** (e.g., support column) of the trajectory frame **100.** As shown in **Figs. 30A** and **30B****,** the EM tracking probe **1500** can comprise a mount **1510** with outwardly extending lugs **1508** that engage the slots **1103** of the guide **1102.** The mount **1510** can include a closed channel or an open channel **1510c** that is sized to slidably snugly hold a (typically rigid or semi-rigid) stem of the tracking probe **1500.** The EM-based tracking system **10EM** can be any suitable system, such as, but not limited to, the StealthStation® AxiEM™ surgical navigation system with electromagnetic (EM) tracking technology by Medtronic, Inc. The EM tracking can use a single-coil or multiple coil design. The at least one coil **1505** can include a coil **1505** that resides at a distal end portion or tip of the probe **1500** or at a location above the distal end of the probe **1500.**

Generally stated, the EM tracking system **10EM** can generate an electromagnetic field around the patient's target anatomy and/or the trajectory frame **100** using a tracking probe **1500** with the at least one EM coil **1505** that can be used to triangulate the position of instruments, e.g., the guide **1102** of the trajectory frame **100** and/or patient-tracking devices during surgical navigation procedures. *See, e.g.,* U.S. Patent No. 8,543,189. EM tracking can be configured so that it does not rely on line-of-sight between the emitter E **(****Fig. 31****)** and the surgical instruments, such as the tracking probe **1500.** The emitter **E** can be draped and kept outside of the sterile field and the staff can move in and out of the EM field with minimal or no disruption to the surgical navigation information. Algorithms of the EM system **10EM** can monitor the electromagnetic field, including metal disturbance, to ensure surgical navigation precision. **Fig. 31** also illustrates that the EM surgical navigation system **10EM** can employ external EM markers **90** on patient anatomy.

The guide **1102** of the trajectory frame **100** can be configured to serially, interchangeably receive the optical and EM tracking/navigation probes **1162, 1190, 1500** to allow for use in different navigation systems.

It is contemplated that a pre-op image of a patient's brain can be imported into the system **10** and displayed on the display with tracking information from the tracking probe **1162, 1190** or **1500.** Patient images can be obtained the day of surgery with the trajectory frame **100** mounted to facilitate registration (aligning orbs or anatomical features between the image sets). The guide **1102** of the trajectory frame **100** can be tracked using the camera or EM system.

In some aspects, for "asleep" procedures, the reference frame **1200** can be attached to a head fixation frame (not shown). For "awake" procedures, the reference frame **1200** can be attached to the trajectory frame as discussed above. CT or other images can be obtained at various points during the procedure, such as at final lead implantation, for example, without requiring constant imaging during a procedure.

Although not shown, in some aspects, one or more of the surgical tools can be configured with one or more lumens and exit ports that deliver desired cellular, biological, and/or drug therapeutics to the target area, such as the brain. The tools may also incorporate transseptal needles, biopsy and/or injection needles as well as ablation means. The lumens, where used, may receive extendable needles that may exit the probe from the distal end or from the sides, proximal, distal, or even, through the electrodes to precisely deliver cellular/biological therapeutics to the desired anatomy target. This delivery configuration may be a potential way to treat patients, where the cellular/biological therapeutics can be delivered into the desired anatomy to modify their cellular function. The cells (*e.g.,* stem cells) may improve function.

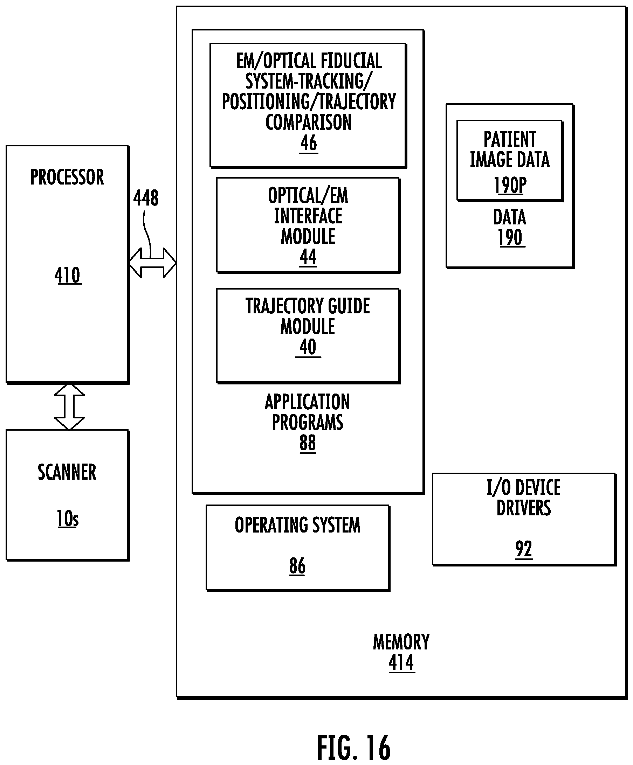

The system **10** can include circuits and/modules that can comprise computer program code used to automatically or semi-automatically carry out operations to generate visualizations and provide output to a user to facilitate image-guided diagnostic and therapy procedures. **Fig. 16** is a schematic illustration of a circuit or data processing system that can be used with the system **10.** The circuits and/or data processing systems may be incorporated in one or more digital signal processors in any suitable device or devices. As shown in **Fig. 16****,** the processor **410** communicates with a scanner **10S** and with memory **414** via an address/data bus **448.** The processor **410** can be any commercially available or custom microprocessor. The memory **414** is representative of the overall hierarchy of memory devices containing the software and data used to implement the functionality of the data processing system. The memory **414** can include, but is not limited to, the following types of devices: cache, ROM, PROM, EPROM, EEPROM, flash memory, SRAM, and DRAM.

As shown in **Fig. 16****,** the memory **414** may include several categories of software and data used in the data processing system: the operating system **86;** the application programs **88;** the input/output (I/O) device drivers **92;** and data **190/190P.** The data can include predefined characteristics of different surgical tools and patient image data. **Fig. 16** also illustrates the application programs **88** can include a Trajectory frame Module **40,** an Interface Module **44** for CLEARPOINT® surgical navigation protocols from MRI Intervention protocols, and an optional Comparison Module **46** (the CLEARPOINT® surgical system protocols can interrogate image data for one or more of a grid patch, a targeting cannula, and a trajectory (guide) frame and/or base and with cooperating defined tools using optical or image fiducials).

As will be appreciated by those of skill in the art, the operating systems **86** may be any operating system suitable for use with a data processing system, such as OS/2, AIX, DOS, OS/390 or System390 from International Business Machines Corporation, Armonk, NY, Windows CE, Windows NT, Windows95, Windows98, Windows2000 or other Windows versions from Microsoft Corporation, Redmond, WA, Unix or Linux or FreeBSD, Palm OS from Palm, Inc., Mac OS from Apple Computer, LabView, or proprietary operating systems. The I/O device drivers **92** typically include software routines accessed through the operating system **86** by the application programs **88** to communicate with devices such as I/O data port(s), data storage and certain memory **84** components. The application programs **88** are illustrative of the programs that implement the various features of the data processing system and can include at least one application, which supports operations according to aspects of the present disclosure. Finally, the data **190** represents the static and dynamic data used by the application programs **88,** the operating system **86,** the I/O device drivers **92,** and other software programs that may reside in the memory **414.**

While the disclosure is illustrated, for example, with reference to the Modules **40, 44, 46** being application programs in **Fig. 16****,** as will be appreciated by those skilled in the art, other configurations may also be utilized For example, the Modules **40, 44, 46** may also be incorporated into the operating system **86,** the I/O device drivers **92** or other such logical division of the data processing system. Thus, the described aspect should not be construed as limited to the configuration of **Fig. 16** which is intended to encompass any configuration capable of carrying out the operations described herein. Further, one or more of modules, *i.e.,* Modules **40, 44, 46** can communicate with or be incorporated totally or partially in other components, such as a workstation, a scanner, an interface device or circuit. Typically, the workstation **30** will include the modules **40, 44, 46** and the scanner or a hospital database or server can have a module that communicates wit the workstation **30** and can provide image data thereto.

The I/O data port can be used to transfer information between the data processing system, the circuit **30c** or workstation **30,** the scanner **10S,** and another computer system or a network (*e.g*., the Internet) or to other devices controlled by or in communication with the processor. These components may be conventional components such as those used in many conventional data processing systems, which may be configured in accordance with the present disclosure to operate as described herein.

The scope of the invention is set forth in the following claims.

## Claims

1. A trajectory frame (100) for use with a surgical system, comprising:
a base (110) having a patient access aperture (112) formed therein, wherein the base is configured to be secured to a body of a patient;
a yoke (120) movably mounted to the base and rotatable about a roll axis;
a platform (130) movably mounted to the yoke and rotatable about a pitch axis;
an elongated guide (1102) secured to the platform, wherein the guide comprises opposite proximal (1102a) and distal (1102b) end portions, wherein the guide distal end portion is positioned proximate the patient access aperture, wherein the guide comprises a bore therethrough that extends from the proximal end portion to the distal end portion, and
at least one device received by the guide (1102) within the bore, wherein the at least one device is removably secured to the guide,
**characterised in that** the at least one device that is received within the bore is an elongate tracking probe mount (1160; 1190m) holding a tracking probe (1162; 1190) with reflective members (1164; 1194) arranged in a fixed geometric relationship relative to each other, and wherein the reflective members are configured to be detectable by a camera-based tracking system (10N/10T)
wherein the tracking probe mount (1160; 1190m) and the trajectory frame (100) comprise image fiducials (50F) detectable in CT and/or MRI images to thereby allow a circuit to calculate an intrabody trajectory using two different methods, one calculated using image interrogation of images with the image fiducials and another calculated using a camera-based system with the reflective members.

2. The trajectory frame of Claim 1, wherein the at least one device further includes a microelectrode probe driver adapter (1170), and wherein the proximal end portion of the guide (1102) is configured to serially, interchangeably and removably secure the tracking probe mount (1160) and the microelectrode probe drive adapter.

3. The trajectory frame of Claim 1, wherein the tracking probe (1162) comprises three spherical reflective members or four spherical reflective members (1164; 1194) in the fixed geometric relationship relative to each other.

4. The trajectory frame of Claim 1, wherein the guide proximal end portion (1102a) comprises at least one longitudinally extending slot (1103) that merges into at least one transversely extending slot (1103a, 1103b), and wherein the tracking probe mount (1160; 1190m) is removably secured within the guide bore via lugs (1168; 1198) extending outwardly therefrom that cooperate with the at least one longitudinally and transversely extending slot to be secured to the guide (1102);
and optionally wherein the at least one device comprises a plurality of devices configured to be releasably and serially inserted within the bore, the devices comprising an elongate tracking probe mount as aforesaid and at least one of the following additional devices: a microelectrode probe driver adapter, a drill guide and drill bit, a skull fixation device and driver, a deep brain stimulation lead guide and a catheter guide.

5. The trajectory frame of Claim 1, wherein the base (110) is configured to be secured to the scalp or skull of a patient about a burr hole formed therein, wherein the guide bore is configured to guide intra-brain placement of at least one device in vivo, and wherein the trajectory frame (100) comprises a bracket (1300) attached thereto that has an arm (1303) that extends outward therefrom and holds a reference frame (1200) that has a plurality of spaced apart reflective fiducials (1194) in a fixed geometry, optionally wherein the platform comprises an X-Y support table movably mounted to the platform that is configured to move in an X-direction and Y-direction relative to the platform, and wherein the guide is secured to the X-Y support table.

6. The trajectory frame of any of Claims 1-4, further comprising:
a plurality of spaced apart ears (1117) held by and extending laterally outward away from the base (110), the ears having upper and lower surfaces; and
a bracket (1300) with a first upright segment (1305) residing under and attached to one ear supporting an arm (1303) that extends outwardly therefrom with a starburst connector (1302) on an end portion of the arm configured to engage a reference frame (1200) with an array of optical fiducials (1204) thereon for tracking by a camera-based tracking system (10N/10T).

7. The trajectory frame of Claim 6, wherein the bracket includes a second upright segment (1305b) residing under and attached to a different ear (1117) and a bridging arm (1306) extending between the first and second upright segments (1305, 1305a).

8. The trajectory frame of Claim 7, wherein the bracket starburst connector (1302) has a first swivel axis that allows positional adjustment of an end portion of the arm, and wherein the first upright segment (1305a) has a starburst connector (1302b) that has a second swivel axis that is orthogonal to the first swivel axis.

9. The trajectory frame of Claim 1, wherein the tracking probe mount (1160; 1190m) comprises a collar (1163; 1192) and stem (1161; 1191s) extending above the collar with a smaller diameter than a body of the tracking probe mount at the collar and extending below the collar, optionally wherein the collar includes an outer wall with at least one aperture (1165) extending therethrough or an upwardly extending protrusion with at least one aperture extending therethrough for cooperating with a fixation member (1166) that extends through the at least one aperture to lock a lower end portion of the array of the tracking probe to the tracking probe mount.

10. The trajectory frame of Claim 1, wherein the base is configured to be secured to the scalp or skull of a patient about a burr hole formed therein, wherein the guide bore is configured to guide intra-brain placement of at least one device in vivo, and wherein the trajectory frame comprises a bracket (1300) attached thereto that has an arm (1303) that extends therefrom and holds a reference frame (1200) with a plurality of spaced-apart reflective members (1204) that are configured to be detectable by a camera (10C) of the camera-based tracking system.

11. The trajectory frame of Claim 1, wherein the elongated guide (1102) secured to the platform is a tubular support column that releasably serially interchangeably holds the plurality of devices.

12. The trajectory frame of Claim 10, wherein the arm (1303) has a length that is between 0.25 inches (6 mm) and 3 inches (76 mm) and rises up as it extends outwardly away from the base (110).

13. The trajectory frame of Claim 1, wherein the tracking probe mount (1160; 1190m) comprises a longitudinally extending through-channel (1169c),

14. A surgical navigation system with a workstation having a User Interface (UI) (44) and a circuit (40) configured to provide a planned trajectory line on an image on a display using the trajectory frame of Claim 1, with reflective members (1164; 1194) detectable by a camera (10C) of a camera-based navigation system (10N/10T) held by the trajectory frame, the circuit UI being configured to provide a user-selectable feature to allow a user to select a second calculation of a planned trajectory line calculated using CT and/or MRI image detectable fiducials (50F) held by or on the trajectory frame to confirm that the planned trajectory line from the camera is proper.

15. The surgical system of Claim 14, wherein the circuit is configured to provide the planned trajectory line on a patient image and/or rendered image using patient image data on the display using the reflective members (1164; 1194) detectable by the camera (10C) of the camera-based navigation system, and wherein the circuit is configured to segment image data to locate the image fiducials for the user selectable second calculation, wherein the image fiducials comprise image fiducials (50F) that are circumferentially spaced apart about the patient access aperture and positioned relative to each other such that at least two are closer together than another to define an affirmative orientation of the trajectory frame, wherein the image fiducials comprise at least three fluid-filled annular MRI and/or CT detectable image fiducials, and wherein the reflective members (1164; 1194) comprise at least three reflective members on the trajectory frame or a tracking probe held by the trajectory frame that are detectable by the camera (10C) of the camera-based navigation system (10N/10T).

## Patentansprüche

1. Ein Bahnrahmen (100) zur Verwendung mit einem chirurgischen System, der Folgendes beinhaltet:
eine Basis (110) mit einer in ihr ausgebildeten Patientenzugangsöffnung (112), wobei die Basis zum Befestigen am Körper eines Patienten konfiguriert ist;
ein Joch (120), das beweglich an der Basis montiert ist und um eine Rollachse drehbar ist;
eine Plattform (130), die beweglich an dem Joch montiert ist und um eine Nickachse drehbar ist;
eine längliche Führung (1102), die an der Plattform befestigt ist, wobei die Führung gegenüberliegende proximale (1102a) und distale (1102b) Endabschnitte beinhaltet, wobei der distale Endabschnitt der Führung in der Nähe der Patientenzugangsöffnung positioniert ist, wobei die Führung eine Bohrung durch sie hindurch beinhaltet, die sich von dem proximalen Endabschnitt bis zu dem distalen Endabschnitt erstreckt, und
mindestens eine Vorrichtung, die von der Führung (1102) innerhalb der Bohrung aufgenommen wird, wobei die mindestens eine Vorrichtung entfernbar an der Führung befestigt ist,
**dadurch gekennzeichnet, dass** die mindestens eine Vorrichtung, die innerhalb der Bohrung aufgenommen wird, eine längliche Nachführsondenhalterung (1160; 1190m) ist, die eine Nachführsonde (1162; 1190) mit reflektierenden Elementen (1164; 1194), die in einem festen geometrischen Verhältnis zueinander angeordnet sind, hält und wobei die reflektierenden Elemente dazu konfiguriert sind, durch ein kamerabasiertes Nachführsystem (10N/10T) detektierbar zu sein,
wobei die Nachführsondenhalterung (1160; 1190m) und der Bahnrahmen (100) Bildreferenzmarker (50F) beinhalten, die in CT- und/oder MRT-Bildern detektierbar sind, um es somit einem Schaltkreis zu erlauben, eine Bahn im Körperinnern unter Verwendung von zwei verschiedenen Methoden zu berechnen, wobei eine unter Verwendung der Bildabfrage von Bildern mit den Bildreferenzmarkern berechnet wird und eine andere unter Verwendung eines kamerabasierten Systems mit den reflektierenden Elementen berechnet wird.

2. Bahnrahmen gemäß Anspruch 1, wobei die mindestens eine Vorrichtung ferner einen Mikroelektrodensonden-Treiberadapter (1170) umfasst und wobei der proximale Endabschnitt der Führung (1102) zum seriellen, auswechselbaren und entfernbaren Befestigen der Nachführsondenhalterung (1160) und des Mikroelektrodensonden-Treiberadapters konfiguriert ist.

3. Bahnrahmen gemäß Anspruch 1, wobei die Nachführsonde (1162) drei kugelförmige reflektierende Elemente oder vier kugelförmige reflektierende Elemente (1164; 1194) in dem festen geometrischen Verhältnis zueinander umfasst.

4. Bahnrahmen gemäß Anspruch 1, wobei der proximale Endabschnitt (1102a) der Führung mindestens einen sich längs erstreckenden Schlitz (1103) beinhaltet, der in mindestens einen sich quer erstreckenden Schlitz (1103a, 1103b) übergeht, und wobei die Nachführsondenhalterung (1160; 1190m) über sich aus dieser nach außen erstreckende Vorsprünge (1168; 1198) entfernbar in der Führungsbohrung befestigt ist, die mit dem mindestens einen sich längs und sich quer erstreckenden Schlitz zum Befestigen an der Führung (1102) zusammenarbeiten;
und wobei die mindestens eine Vorrichtung optional eine Vielzahl von Vorrichtungen beinhaltet, die dazu konfiguriert sind, lösbar und seriell in die Bohrung einführbar zu sein, wobei die Vorrichtungen eine längliche Nachführsondenhalterung wie zuvor dargestellt und mindestens eine der folgenden zusätzlichen Vorrichtungen beinhalten: einen Mikroelektrodensonden-Treiberadapter, eine Bohrführung und einen Bohrmeißel, eine Schädelfixiervorrichtung und einen Treiber, eine Tiefenhirn-Stimulationskabelführung und eine Katheterführung.

5. Bahnrahmen gemäß Anspruch 1, wobei die Basis (110) dazu konfiguriert ist, an der Kopfhaut oder dem Schädel eines Patienten um ein darin gebildetes Bohrloch befestigt zu werden, wobei die Führungsbohrung dazu konfiguriert ist, die innerhalb des Gehirns erfolgende Platzierung mindestens einer Vorrichtung in vivo zu führen, und wobei der Bahnrahmen (100) einen daran angebrachten Träger (1300) beinhaltet, der einen Arm (1303) aufweist, der sich aus diesem nach außen erstreckt und einen Bezugsrahmen (1200) hält, der eine Vielzahl von beabstandeten reflektierenden Referenzmarkern (1194) in einer festen Geometrie aufweist, wobei die Plattform optional einen beweglich an der Plattform montierten X-Y-Auflagetisch beinhaltet, der zur Bewegung in einer X-Richtung und Y-Richtung relativ zu der Plattform konfiguriert ist, und wobei die Führung an dem X-Y-Auflagetisch befestigt ist.

6. Bahnrahmen gemäß einem der Ansprüche 1-4, der ferner Folgendes beinhaltet:
eine Vielzahl von beabstandeten Ohren (1117), die von der Basis (110) gehalten werden und sich seitlich von dieser weg erstrecken, wobei die Ohren obere und untere Oberflächen aufweisen; und
einen Träger (1300) mit einem ersten aufrechten Segment (1305), das sich unter einem Ohr befindet und an einem Ohr angebracht ist, das einen Arm (1303) stützt, der sich von diesem nach außen erstreckt, wobei ein sternförmiger Steckverbinder (1302) an einem Endabschnitt des Arms zur Eingriffnahme eines Bezugsrahmens (1200) mit einer Anordnung optischer Referenzmarker (1204) zum Nachführen durch ein kamerabasiertes Nachführsystem (10N/10T) konfiguriert ist.

7. Bahnrahmen gemäß Anspruch 6, wobei der Träger ein zweites aufrechtes Segment (1305b), das sich unter einem anderen Ohr (1117) befindet und an diesem angebracht ist, und einen Überbrückungsarm (1306), der sich zwischen dem ersten und zweiten aufrechten Segment (1305, 1305a) erstreckt, umfasst.

8. Bahnrahmen gemäß Anspruch 7, wobei der sternförmige Steckverbinder (1302) des Trägers eine erste Schwenkachse aufweist, die eine Positionseinstellung eines Endabschnitts des Arms erlaubt, und wobei das erste aufrechte Segment (1305a) einen sternförmigen Steckverbinder (1302b) aufweist, der eine zweite Schwenkachse aufweist, die im rechten Winkel zu der ersten Schwenkachse steht.

9. Bahnrahmen gemäß Anspruch 1, wobei die Nachführsondenhalterung (1160; 1190m) einen Bund (1163; 1192) und einen sich oberhalb des Bunds erstreckenden Stiel (1161; 1191s) mit einem kleineren Durchmesser als ein Körper der Nachführsondenhalterung an dem Bund der sich unterhalb des Bunds erstreckt, beinhaltet, wobei der Bund optional eine Außenwand mit mindestens einer Öffnung (1165), die sich durch diese erstreckt, oder eine sich nach oben erstreckende Auskragung mit mindestens einer sich dadurch erstreckende Öffnung zum Kooperieren mit einem Fixierungselement (1166) umfasst, das sich durch die mindestens eine Öffnung erstreckt, um einen unteren Endabschnitt der Anordnung der Nachführsonde mit der Nachführsondenhalterung zu arretieren.

10. Bahnrahmen gemäß Anspruch 1, wobei die Basis dazu konfiguriert ist, an der Kopfhaut oder dem Schädel eines Patienten um ein darin gebildetes Bohrloch befestigt zu werden, wobei die Führungsbohrung dazu konfiguriert ist, die innerhalb des Gehirns erfolgende Platzierung mindestens einer Vorrichtung in vivo zu führen, und wobei der Bahnrahmen einen daran angebrachten Träger (1300) beinhaltet, der einen Arm (1303) aufweist, der sich aus diesem erstreckt und einen Bezugsrahmen (1200) mit einer Vielzahl von beabstandeten reflektierenden Elementen (1204) hält, die dazu konfiguriert sind, durch eine Kamera (10C) des kamerabasierten Nachführsystems detektierbar zu sein.

11. Bahnrahmen gemäß Anspruch 1, wobei die längliche Führung (1102), die an der Plattform befestigt ist, eine röhrenförmige Tragsäule ist, die die Vielzahl von Vorrichtungen lösbar, seriell, austauschbar hält.

12. Bahnrahmen gemäß Anspruch 10, wobei der Arm (1303) eine Länge aufweist, die zwischen 0,25 Zoll (6 mm) und 3 Zoll (76 mm) beträgt und die zunimmt, wenn er sich von der Basis (110) nach außen bewegt.

13. Bahnrahmen gemäß Anspruch 1, wobei die Nachführsondenhalterung (1160; 1190m) einen sich längs erstreckenden Durchgangskanal (1169c) beinhaltet.

14. Ein chirurgisches Navigationssystem mit einer Arbeitsstation, die eine Benutzerschnittstelle (UI) (44) und einen Schaltkreis (40) aufweist, dazu ausgelegt, eine geplante Bahnlinie auf einem Bild auf einer Anzeige unter Verwendung des Bahnrahmens gemäß Anspruch 1 bereitzustellen, mit reflektierenden Elementen (1164; 1194), die durch eine Kamera (10C) eines kamerabasierten Navigationssystems (10N/10T), das von dem Bahnrahmen gehalten wird, detektierbar sind, wobei der Schaltkreis UI dazu konfiguriert ist, ein vom Benutzer auswählbares Merkmal bereitzustellen, um es einem Benutzer zu erlauben, eine zweite Berechnung einer geplanten Bahnlinie auszuwählen, die unter Verwendung von in CT- und/oder MRT-Bildern detektierbaren Referenzmarkern (50F) berechnet wurde, die von dem oder auf dem Bahnrahmen gehalten werden, um zu bestätigen, dass die geplante Bahnlinie von der Kamera richtig ist.

15. Chirurgisches System gemäß Anspruch 14, wobei der Schaltkreis dazu konfiguriert ist, die geplante Bahnlinie auf einem Patientenbild und/oder gerenderten Bild unter Verwendung von Patientenbilddaten auf der Anzeige unter Verwendung der reflektierenden Elemente (1164; 1194), die durch die Kamera (10C) des kamerabasierten Navigationssystems detektierbar sind, bereitzustellen, und wobei der Schaltkreis dazu konfiguriert ist, Bilddaten zu segmentieren, um die Bildreferenzmarker für die vom Benutzer auswählbare zweite Berechnung zu lokalisieren, wobei die Bildreferenzmarker Bildreferenzmarker (50F) beinhalten, die umfangsmäßig um die Patientenzugangsöffnung beabstandet sind und relativ zueinander positioniert sind, sodass mindestens zwei näher zusammen sind als ein anderer, um eine affirmative Ausrichtung des Bahnrahmens zu bestätigen, wobei die Bildreferenzmarker mindestens drei mit Fluid gefüllte ringförmige MRT- und/oder CT-detektierbare Bildreferenzmarker beinhalten, und wobei die reflektierenden Elemente (1164; 1194) mindestens drei reflektierende Elemente auf dem Bahnrahmen oder einer Nachführsonde, die von dem Bahnrahmen gehalten wird, beinhalten, die durch die Kamera (10C) des kamerabasierten Navigationssystems (10N/10T) detektierbar sind.

## Revendications

1. Cadre de trajectoire (100) destiné à être utilisé avec un système chirurgical, comprenant :
une base (110) qui comprend une ouverture d'accès au patient (112) formée dans celle-ci, la base étant conçue pour être fixée au corps d'un patient ;
une fourche (120) montée de manière amovible à la base et rotative autour d'un axe de roulis ;
une plateforme (130) montée de manière amovible à la fourche et rotative autour d'un axe de roulis ;
un guide allongé (1102) fixé à la plateforme, dans lequel le guide comprend des parties d'extrémité proximale (1102a) et distale (1102b) opposées, dans lequel la partie d'extrémité distale du guide est positionnée à proximité de l'ouverture d'accès au patient, dans lequel le guide comprend un alésage le traversant et qui s'étend de la partie d'extrémité proximale à la partie d'extrémité distale, et
au moins un dispositif reçu par le guide (1102) à l'intérieur de l'alésage, dans lequel le au moins un dispositif est fixé de manière amovible au guide,
**caractérisé en ce que** le au moins un dispositif qui est reçu à l'intérieur de l'alésage est un support de sonde de suivi allongé (1160 ; 1190m) tenant une sonde de suivi (1162 ; 1190) avec des éléments réfléchissants (1164 ; 1194) placés dans une relation géométrique fixe les uns par rapport aux autres, et dans lequel les éléments réfléchissants sont conçus pour être détectables par un système de suivi à base de caméra (10N/10T)
dans lequel le support de sonde de suivi (1160; 1190 m) et le cadre de trajectoire (100) comprennent des clichés d'imagerie (50F) détectables dans des images de TDM et/ou d'IRM pour permettre ainsi à un circuit de calculer une trajectoire intracorporelle utilisant deux procédés différents, un calculé en utilisant une interrogation de l'image des images avec les clichés d'imagerie et un autre calculé en utilisant un système à base de caméra avec des éléments réfléchissants.

2. Cadre de trajectoire selon la revendication 1, dans lequel le au moins un dispositif comprend en outre un adaptateur d'entraînement de sonde à microélectrode (1170), et dans lequel la partie d'extrémité proximale du guide (1102) est conçue pour fixer en série, de manière interchangeable et amovible le support de sonde de suivi (1160) et l'adaptateur d'entraînement de sonde à microélectrodes.

3. Cadre de trajectoire selon la revendication 1, dans lequel la sonde de suivi (1162) comprend trois éléments réfléchissants sphériques ou quatre éléments réfléchissants sphériques (1164 ; 1194) dans la relation géométrique fixe l'un par rapport à l'autre.

4. Cadre de trajectoire selon la revendication 1, dans lequel la partie d'extrémité proximale du guide (1102a) comprend au moins une fente s'étendant longitudinalement (1103) qui fusionne dans au moins une fente s'étendant transversalement (1103a, 1103b), et dans lequel le support de sonde de suivi (1160 ; 1190m) est fixé de manière amovible dans l'alésage du guide par des ergots (1168 ; 1198) s'étendant vers l'extérieur de celui-ci qui coopère avec la au moins une fente s'étendant longitudinalement et transversalement pour être fixée au guide (1102) ;
et éventuellement dans lequel le au moins un dispositif comprend une pluralité de dispositifs conçus pour être insérés de manière libérable et en série dans l'alésage, les dispositifs comprenant un support de guide de suivi allongé comme susmentionné et au moins un des dispositifs supplémentaires suivants : un adaptateur d'entraînement de sonde de microélectrode, un guide-foret et un trépan de forage, un dispositif de fixation de crâne et un moyen d'entraînement, un guide d'électrode de stimulation cérébrale profonde et un guide de cathéter.

5. Cadre de trajectoire selon la revendication 1, dans lequel la base (110) est conçue pour être fixée au cuir chevelu ou au crâne d'un patient autour d'un trou de trépan qui y est formé, dans lequel l'alésage de guidage est conçu pour guider un placement intracérébral d'au moins un dispositif *in vivo,* et dans lequel le cadre de trajectoire (100) comprend un support (1300) qui y est fixé et qui a un bras (1303) qui s'étend vers l'extérieur de celui-ci et tient un cadre de référence (1200) qui a une pluralité de clichés réfléchissants espacés (1194) dans une géométrie fixe, éventuellement dans lequel la plateforme comprend une table de support X-Y montée de manière amovible à la plateforme qui est conçue pour se déplacer dans une direction X et une direction Y par rapport à la plateforme, et dans lequel le guide est fixé à la table de support X-Y.

6. Cadre de trajectoire selon l'une quelconque des revendications 1 à 4, comprenant en outre :
une pluralité d'oreilles espacées (1117) tenues par et s'étendant latéralement vers l'extérieur en s'éloignant de la base (110), les oreilles ayant des surfaces supérieures et inférieures ; et
un support (1300) avec un premier segment vertical (1305) résidant sous et fixé à une oreille supportant un bras (1303) qui s'étend vers l'extérieur depuis celui-ci avec un connecteur étoilé (1302) sur une partie d'extrémité du bras conçue pour mettre en prise un cadre de référence (1200) avec un ensemble de clichés optiques (1204) dessus pour suivre par un système de suivi à base de caméra (10N/10T).

7. Cadre de trajectoire selon la revendication 6, dans lequel le support comprend un second segment vertical (1305b) résidant sous et fixé à une oreille différente (1117) et un bras de pontage (1306) s'étendant entre les premier et second segments verticaux (1305, 1305a).

8. Cadre de trajectoire selon la revendication 7, dans lequel le connecteur étoilé de support (1302) a un premier axe pivotant qui permet un réglage de position d'une partie d'extrémité du bras, et dans lequel le premier segment vertical (1305a) a un connecteur étoilé (1302b) qui a un second axe pivotant qui est orthogonal au premier axe pivotant.

9. Cadre de trajectoire selon la revendication 1, dans lequel le support de sonde de suivi (1160 ; 1190m) comprend un collet (1163 ; 1192) et une tige (1161 ; 1191s) s'étendant au-dessus du collet avec un plus petit diamètre qu'un corps du support de guide de suivi au collet et s'étendant sous le collet, éventuellement dans lequel le collet comprend une paroi extérieure avec au moins une ouverture (1165) s'y étendant ou une saillie s'étendant verticalement avec au moins une ouverture s'y étendant pour coopérer avec un élément de fixation (1166) qui s'étend à travers la au moins une ouverture pour verrouiller une partie d'extrémité inférieure du réseau de la sonde de suivi au support de sonde de suivi.

10. Cadre de trajectoire selon la revendication 1, dans lequel la base est conçue pour être fixée au cuir chevelu ou au crâne d'un patient autour d'un trou de trépan qui y est formé, dans lequel l'alésage de guidage est conçu pour guider un placement intracérébral d'au moins un dispositif *in vivo,* et dans lequel le cadre de trajectoire comprend un support (1300) qui y est fixé et qui a un bras (1303) qui s'y étend et tient un cadre de référence (1200) avec une pluralité d'éléments réfléchissants espacés (1204) qui sont conçus pour être détectables par une caméra (10C) du système de suivi à base de caméra.

11. Cadre de trajectoire selon la revendication 1, dans lequel le guide allongé (1102) fixé à la plateforme est une colonne de support tubulaire qui tient de manière interchangeable, en série et de manière libérable la pluralité de dispositifs.

12. Cadre de trajectoire selon la revendication 10, dans lequel le bras (1303) a une longueur qui varie entre 0,25 po (6 mm) et 3 po (76 mm) et qui monte alors qu'il s'étend vers l'extérieur en s'éloignant de la base (110).

13. Cadre de trajectoire selon la revendication 1, dans lequel le support de sonde de suivi (1160; 1190m) comprend un canal traversant s'étendant longitudinalement (1169c).

14. Système chirurgical de navigation avec un poste de travail ayant une interface d'utilisateur (IU) (44) et un circuit (40) conçu pour fournir une ligne de trajectoire prévue sur une image sur un écran utilisant le cadre de trajectoire selon la revendication 1, avec des éléments réfléchissants (1164 ; 1194) détectables par une caméra (10C) d'un système de navigation à base de caméra (10N/10T) tenu par le cadre de trajectoire, l'interface d'utilisateur de circuit étant conçue pour fournir une caractéristique pouvant être sélectionnée par un utilisateur pour permettre à un utilisateur de sélectionner un second calcul d'une ligne de trajectoire prévue calculée en utilisant des clichés détectables (50F) d'une image de TMD et/ou d'IRM tenus par ou sur le cadre de trajectoire pour confirmer que la ligne de trajectoire prévue de la caméra est appropriée.

15. Système chirurgical selon la revendication 14, dans lequel le circuit est conçu pour offrir la ligne de trajectoire prévue sur une image d'un patient et/ou une image rendue en utilisant les données d'image d'un patient sur l'écran en utilisant les éléments réfléchissants (1164 ; 1194) détectables par la caméra (10C) du système de navigation à base de caméra, et dans lequel le circuit est conçu pour segmenter des données d'image pour localiser les clichés d'image pour le second calcul sélectionnable par un utilisateur, dans lequel les clichés d'images comprennent des clichés d'images (50F) qui sont espacés circonférentiellement autour de l'ouverture d'accès d'un patient et positionnés les uns par rapport aux autres de sorte qu'au moins deux sont plus près qu'un autre pour définir une orientation affirmative du cadre de trajectoire, dans lequel les clichés d'image comprennent au moins trois clichés d'image détectables par IRM et/ou TMD annulaire rempli de fluide, et dans lequel les éléments réfléchissants (1164 ; 1194) comprennent au moins trois éléments réfléchissants sur le cadre de trajectoire ou une sonde de suivi tenue par le cadre de trajectoire qui sont détectables par la caméra (10C) du système de navigation à base de caméra (10N/10T).
